(19) Europäisches Patentamt
European Patent Office
Office européen des brevets

(11) **EP 2 056 937 B1**

(12) **FASCICULE DE BREVET EUROPEEN**

(45) Date de publication et mention de la délivrance du brevet:
**29.03.2017 Bulletin 2017/13**

(21) Numéro de dépôt: **07788387.4**

(22) Date de dépôt: **13.08.2007**

(51) Int Cl.:
*A61N 7/02* *(2006.01)*     *A61B 34/10* *(2016.01)*
*A61B 90/00* *(2016.01)*

(86) Numéro de dépôt international:
**PCT/EP2007/058361**

(87) Numéro de publication internationale:
**WO 2008/025667 (06.03.2008 Gazette 2008/10)**

(54) **DISPOSITIF DE TRAITEMENT VOLUMIQUE DE TISSUS BIOLOGIQUES**

VORRICHTUNG ZUR VOLUMENBEHANDLUNG VON BIOLOGISCHEM GEWEBE

DEVICE FOR THE VOLUME TREATMENT OF BIOLOGICAL TISSUE

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **29.08.2006 FR 0607584**

(43) Date de publication de la demande:
**13.05.2009 Bulletin 2009/20**

(73) Titulaire: **Koninklijke Philips N.V.**
**5656 AE Eindhoven (NL)**

(72) Inventeurs:
• **MOONEN, Chrétien**
  **33170 Gradignan (FR)**
• **MOUGENOT, Charles**
  **33000 Bordeaux (FR)**

(74) Mandataire: **Damen, Daniel Martijn et al**
**Philips Intellectual Property & Standards**
**High Tech Campus 5**
**5656 AE Eindhoven (NL)**

(56) Documents cités:
WO-A-02/43804     WO-A-02/085457
FR-A1- 2 869 548     US-A- 6 128 522

**Description**

DOMAINE DE L'INVENTION

L'invention concerne le domaine du traitement de tissus biologiques par hyperthermie.

ETAT DE LA TECHNIQUE

Les thérapies par hyperthermie locale consistent à chauffer, localement, une zone ou région cible d'un tissu biologique. Lorsque ce type de thérapie est utilisé dans le cadre de la thérapie génique la chaleur peut-être, par exemple, utilisée pour son action sur un promoteur thermosensible. On peut également utiliser la chaleur pour nécroser des tissus biologiques et procéder à l'ablation de tumeurs.

Aussi, les thérapies par hyperthermie locale offrent de nombreux avantages. Ces avantages sont aussi bien qualitatifs qu'économiques. Du point de vue qualitatif, elles offrent, par exemple, un fort potentiel pour le contrôle de traitements tels que les thérapies géniques, le dépôt local de médicaments, l'ablation de tumeurs. Du point de vue économique, elles sont compatibles avec un traitement ambulatoire des malades, elles permettent de réduire les durées d'hospitalisation, etc.

Dans les thérapies par hyperthermie, la chaleur peut être, par exemple, apportée par un laser, des micro-ondes ou des ondes radiofréquences, des ultrasons focalisés, etc. D'une manière générale, les thérapies par hyperthermie locale permettent des interventions médicales dont la nature invasive est réduite au minimum. Mais parmi les types d'énergie précités, les ultrasons focalisés sont particulièrement intéressants puisqu'ils permettent de chauffer la zone de focalisation, de manière non invasive, profondément dans un corps biologique, sans chauffer de manière significative les tissus voisins de la zone de focalisation.

Dans tous les cas, la température de la zone et de son environnement immédiat, pendant le traitement, doit être contrôlée précisément et de manière continue. A cette fin, on peut utiliser l'Imagerie par Résonance Magnétique (IRM). L'IRM permet, en effet, d'obtenir une cartographie précise des distributions de températures ainsi que des informations anatomiques détaillées. L'IRM permet en outre, par un asservissement de l'outil de chauffage, un contrôle non invasif de la température et rend donc, dans le cas des ultrasons, le dispositif de traitement totalement non invasif.

On connaît déjà des dispositifs pour le contrôle ponctuel de la température, pendant les traitements par des ultrasons focalisés, basés sur la thermométrie par résonance magnétique.

La taille des tumeurs à traiter est en général bien plus grande que la taille du point focal. À la fréquence de 1,5MHz la taille du point focal à une dimension proche de la longueur d'onde, soit de 1 mm dans les organes biologiques. En revanche les tumeurs cancéreuses décelables par IRM sont plus proches du centimètre. Bien qu'un traitement chimio thérapeutique permette de réduire la dimension de ses tumeurs, un traitement ultrasonore ponctuel est insuffisant.

La technique de contrôle ponctuel de la température peut-être utilisée successivement sur plusieurs points. Toutefois ce protocole est très long car il faut attendre plusieurs minutes entre chaque point pour que la température retourne à l'état initial. De plus il subsiste un risque que des cellules tumorales se trouvant entre deux points cibles ne soient pas nécrosées.

Il a été ensuite envisagé de réaliser un contrôle spatial de la température, qui utilise une technique similaire au contrôle de la température ponctuel, seulement plusieurs points sont chauffés simultanément. Une solution a par exemple été proposée dans la demande de brevet français n° 04-04562 déposée le 24 avril 2004 et intitulée « Ensemble de traitement thermique de tissus biologiques », permettant un contrôle de la température selon un plan spécifique de la région cible. Une telle solution impose néanmoins des contraintes directionnelles importantes pour la focalisation, et requiert en outre des temps de traitement longs, rendant le dispositif peu efficace. Un autre système proposé dans la demande PCT publiée sous le numéro WO 02/43804 propose de suivre la distribution de la dose thermique associée à plusieurs points de traitement, en vue de choisir les positions des points de traitement ultérieurs de manière à traiter les zones non encore nécrosées, les paramètres de dose thermique étant généralement constants. Si un tel système permet d'éviter de traiter des zones ayant déjà atteint la dose thermique suffisante pour une nécrose, il présente un certains nombre d'inconvénients. En effet, il est d'abord très imprécis puisque le suivi de la distribution de la dose thermique est relativement binaire, c'est à dire qu'il permet uniquement de déterminer si une zone est nécrosée ou non, pour savoir s'il ladite zone doit encore être traitée. En outre, le suivi de la distribution de la dose thermique implique des contraintes de fonctionnement importantes tendant à ralentir le processus de traitement, puisqu'il est notamment nécessaire d'opérer un refroidissement complet de la région traitée avant d'avoir une image fidèle de la dose thermique atteinte.

Un but de la présente invention est de fournir un dispositif de traitement thermique d'une région cible, et un procédé associé, qui permet de résoudre au moins l'un des inconvénients précités.

EXPOSE DE L'INVENTION

A cette fin, on propose un dispositif de traitement thermique tel que défini à la revendication 1.

Un tel dispositif permet d'obtenir le traitement d'un large volume, qui peut être tridimensionnel, et ceci avec très grande homogénéité.

En effet, lors de la focalisation de la source d'énergie sur un point de traitement ponctuel, il s'ensuit une répartition spatiale d'énergie qui n'est pas uniquement localisée sur le point de traitement mais qui s'étend également aux zones voisines du point de traitement. Ainsi, la prise en compte des points de traitements antérieurs permet d'ajuster la position et l'intensité énergétique du nouveau point de traitement à effectuer, de façon à ne pas surexposer une zone de la région cible.

On évite donc les chevauchements indésirés de traitements ponctuels qui peuvent générer des surexpositions. En outre, le dispositif proposé est très précis puisqu'il permet le paramétrage de chaque point de traitement à venir en fonction de la répartition spatiale d'énergie de chaque point de traitement antérieur. La distribution spatiale de température dans la région permet en effet de déduire les répartitions spatiales d'énergies des points de traitement antérieurs pour en déduire la répartition spatiale d'énergie restant à effectuer en vue du traitement complet de la région. Ceci est particulièrement avantageux dans le cas où le seuil de nécrose de la région cible doit être atteint sans être vraiment dépassé comme cela est le cas dans le cadre d'un suivi relativement binaire de la dose thermique. De plus, il n'est pas nécessaire d'effectuer un quelconque refroidissement de la région cible pour suivre la distribution de température, ce qui permet d'optimiser le temps de traitement.

Enfin, la prise en compte de l'effet de chevauchement des répartitions spatiales d'énergies permet de réduire significativement l'énergie totale utilisée pour traiter une région, et donc les risques associés pour le patient, puisque les points de traitements localisés au centre de cette région ne sont alors plus nécessaires.

Des aspects préférés mais non limitatifs de ce dispositif de traitement thermique sont les suivants :

- la répartition spatiale des points de traitement est tridimensionnelle ;
- l'unité de contrôle comprend des moyens pour déterminer la répartition spatiale et énergétique des points de traitement à effectuer en fonction d'une répartition spatiale d'énergie requise définie par un système de régulation de la température pour traiter la région cible ;
- l'unité de contrôle comprend des moyens pour déterminer la répartition spatiale d'énergie requise pour traiter la région cible selon un système de régulation Proportionnel-Intégral-Dérivé ;
- les moyens pour déterminer la répartition spatiale et énergétique des points de traitement à effectuer comprennent des moyens de déconvolution de la répartition spatiale d'énergie requise pour traiter la région cible par une répartition spatiale d'énergie caractéristique d'un point de traitement ponctuel ;
- les moyens pour déterminer la répartition spatiale et énergétique des points de traitement à effectuer comprennent des moyens pour déterminer le point de traitement correspondant au maximum d'une répartition spatiale d'énergie restante pour le traitement de la région cible, la répartition spatiale d'énergie restante correspondant à la répartition spatiale d'énergie requise pour traiter la région cible retranchée des répartitions spatiales d'énergie caractéristiques des points de traitement antérieurs ;
- l'unité de contrôle comprend des moyens pour déterminer la répartition spatiale et énergétique des points de traitement à effectuer tel que :

$$E^0(\vec{r}) = E^{REQUISE}(\vec{r}) \quad \text{et} \quad \Gamma^0(\vec{r}) = 0$$

$$\left|
\begin{aligned}
&\text{soit } \vec{r}_n \text{ tel que } E^n(\vec{r}_n) = \max(E^n(\vec{r})) \\
&E^{n+1}(\vec{r}) = E^n(\vec{r}) - R \cdot E^n(\vec{r}_n) \cdot E^{1pt}(\vec{r} - \vec{r}_n) \\
&\Gamma^{n+1}(\vec{r}) = \Gamma^n(\vec{r}) + R \cdot E^n(\vec{r}_n) \cdot \delta(\vec{r} - \vec{r}_n)
\end{aligned}
\right.$$

où $E^i(\vec{r})$ correspond à la différence entre la répartition spatiale d'énergie requise et les répartitions spatiales d'énergie caractéristiques des $i$ points de traitement $\vec{r}_i$ antérieurs, $\Gamma^i(\vec{r})$ correspond à la répartition spatiale des points de traitement associés, $\delta$ est la fonction de Dirac qui est nulle en tout point $\vec{r}$ différent de l'origine où elle vaut 1, $R$ est un pourcentage choisi de façon arbitraire pour que la répartition spatiale d'énergie caractéristique d'un point de traitement soit inférieure à la répartition spatiale d'énergie requise $E^{REQUISE}(\vec{r})$,

- l'unité de contrôle comprend des moyens pour contrôler l'énergie fournie par les moyens générateurs d'énergie en fonction d'une consigne de température non uniforme.

- l'unité de contrôle comprend des moyens pour contrôler l'énergie fournie par les moyens générateurs d'énergie en fonction d'une consigne de température décalée temporellement pour chaque point de traitement.

On propose également un procédé de traitement thermique d'une région cible d'un tissu biologique comprenant les étapes consistant à :

- mesurer la distribution spatiale de température dans ladite région,
- commander le déplacement d'un point focal (P) vers des points de traitement successifs dans la région cible,
- fournir de l'énergie au point focal (P),

caractérisé en ce qu'il comprend en outre l'étape consistant à déterminer une répartition spatiale et énergétique des points de traitement à effectuer, chaque point de traitement successif ayant une position et une énergie de traitement déterminée en fonction des positions et répartitions spatiales d'énergies des points de traitement antérieurs.

Le procédé de traitement proposé peut être mis en oeuvre par les moyens du dispositif de traitement proposé plus haut. Ce dispositif de traitement comprend en outre des moyens permettant la mise en oeuvre d'autres étapes caractéristiques du procédé de traitement proposé selon des aspects préférés et non limitatifs.

DESCRIPTION DES FIGURES

D'autres caractéristiques et avantages ressortiront encore de la description qui suit, laquelle est purement illustrative et non limitative et doit être lue en regard des figures annexées parmi lesquelles :

- La figure 1 illustre les différents éléments caractéristiques du dispositif de traitement thermique ;
- Les figures 2a à 2c illustrent le déphasage des signaux électriques pour déplacer le point focal électroniquement ;
- La figure 3 représente la répartition des 256 éléments sur la face avant d'un transducteur matriciel ;
- Les figures 4a et 4b illustrent une simulation du champ acoustique décrivant la forme du point focal, c'est à dire la répartition spatiale d'énergie induite par un point de traitement ;
- La figure 5 illustre l'architecture d'un générateur de 256 signaux électriques ;
- Les figures 6a à 6c présentent des cartes de température obtenues en déplaçant électroniquement le point focal en quatre points ;
- La figure 7 représente une succession de cartes de température obtenues en déplaçant électroniquement le point focal selon deux spirales successives ;
- Les figures 8a à 8c illustrent la transformé de Fourrier de la forme du point focal, c'est à dire de la répartition spatiale d'énergie induite par un point de traitement, dans trois plans orthogonaux, respectivement le plan $k_Z k_Y$, le plan $k_X k_Z$, et le plan $k_X k_Y$ ;
- La figure 9 illustre le dépassement de l'énergie produite par rapport à l'énergie requise à cause du chevauchement des points de focalisations ;
- Les figures 10a à 10c illustrent les trois premières itérations de l'algorithme de détection du maximum ;
- La figure 11 illustre l'énergie produite par rapport à l'énergie requise obtenue avec les trois premières itérations de l'algorithme de détection du maximum ;
- Les figures 12 à 15 illustrent la mise en oeuvre de l'algorithme de détection de maximum établissant une densité de points (figures 14i, 14ii, et 14iii) et l'énergie produite (figures 15i, 15ii, et 15iii) par cette densité de points pour une répartition cubique de l'énergie requise (figures 13i, 13ii, et 13iii) et une forme de point focale donnée ((figures 12i, 12ii, et 12iii) ; les indices i, ii, et iii caractérisant les différentes figures indiquent que l'illustration est faite respectivement selon le plan transversal, le plan coronal, et le plan saggital ;
- Les figures 16a à 16c représentent la densité de points établie par l'algorithme de détection de maximum sur trois coupes centrales pour une répartition cubique de l'énergie requise ;
- La figure 17 représente la trajectoire de 12 points extraits de la densité de points des figures 16a à 16c ;
- Les figures 18a et 18b illustrent la consigne de température temporelle et spatiale décalée de 5s/mm pour les points excentrés ;
- Les figures 19a et 19b représentent deux cartes thermiques obtenue durant un contrôle de la température sur un segment de 11 mm de largeur ;
- La figure 19c représente une carte de dose thermique obtenue après un contrôle de la température sur un segment de 11 mm de largeur ;
- La figure 20 représente la température maximum, moyenne et minimum au cours du temps pour les 11 voxels asservis comme aux figures 19a et 19b par rapport à la température de consigne ;
- Les figures 21 a et 21 b illustrent la répartition spatiale de la température le long des axes X et Z respectivement, au début, au milieu et à la fin de la montée en température faite comme aux figures 19a et 19b ;

- Les figures 22a et 22b illustrent la répartition spatiale de la température le long des axes X et Z respectivement, à 4 instants durant le maintien de la température à 15°C du chauffage des figures 19a et 19b ;
- Les figures 23a et 23b représentent deux cartes thermiques obtenue durant un contrôle de la température sur un segment de 11 mm de largeur avec un décalage de la consigne de température pour les points excentrés ;
- La figure 23c représente une carte de dose thermique obtenue après un contrôle de la température sur un segment de 11 mm de largeur avec un décalage de la consigne de température pour les points excentrés ;
- La figure 24 représente la température maximum, moyenne et minimum au cours du temps pour les 11 voxels asservis comme aux figures 23a et 23b par rapport à la température de consigne ;
- Les figures 25a et 25b illustrent la répartition spatiale de la température le long des axes X et Z respectivement, à 5 instants différents de la montée en température faite comme aux figures 23a et 23b ;
- Les figures 26a et 26b illustrent la répartition spatiale de la température le long des axes X et Z respectivement, à 4 instants durant le maintien de la température à 15°C du chauffage des figures 23a et 23b ;
- La figure 27 représente la largeur à mi-hauteur, soit 7.5°C, des chauffages des figures 19a et 19b, et 23a et 23b réalisés avec une température de consigne synchronisée ou décalée en chaque point ;
- Les figures 28 et 29 représentent des cartes thermiques respectivement au milieu et à la fin de la montée en température, et les figures 30 représentent les cartes de dose thermique obtenue après un contrôle de la température sur volume cubique de $8\times8\times12mm^3$ ; les indices i, ii, et iii caractérisant les différentes figures indiquent que l'illustration est faite respectivement selon la coupe 3, la coupe 4, et la coupe 5 respectivement ;
- La figure 31 représente la température maximum, moyenne et minimum au cours du temps pour les 147 voxels asservis comme aux figures 28 et 29 par rapport à la température de consigne ;
- Les figures 32 et 33 représentent la répartition spatiale de la température le long des axes X et Z sur trois coupes au début, au milieu et à la fin de la montée en température du chauffage des figures 28 et 29 ; les indices i, ii, et iii caractérisant les différentes figures indiquent que l'illustration est faite respectivement selon la coupe 3, la coupe 4, et la coupe 5 respectivement ;
- La figure 34 représente la température le long de l'axe Y au début, au milieu et à la fin de la montée en température du chauffage des figures 28 et 29 ;
- Les figures 35 et 36 représentent des cartes thermiques respectivement au milieu et à la fin de la montée en température, et les figures 37 représentent les cartes de dose thermique obtenue après un contrôle de la température sur volume elliptique de $8\times8\times12mm^3$ ; les indices i, ii, et iii caractérisant les différentes figures indiquent que l'illustration est faite respectivement selon la coupe 3, la coupe 4, et la coupe 5 respectivement ;
- La figure 38 représente la température maximum, moyenne et minimum au cours du temps pour les 87 voxels asservis comme aux figures 35 et 36 par rapport à la température de consigne ;
- Les figures 39 et 40 représentent la répartition spatiale de la température le long des axes X et Z sur trois coupes au début, au milieu et à la fin de la montée en température du chauffage des figures 35 et 36 ; les indices i, ii, et iii caractérisant les différentes figures indiquent que l'illustration est faite respectivement selon la coupe 3, la coupe 4, et la coupe 5 respectivement ;
- La figure 41 représente la température le long de l'axe Y au début, au milieu et à la fin de la montée en température du chauffage des figures 35 et 36.

DESCRIPTION DETAILLEE DE L'INVENTION

**Description Matériel**

Description globale

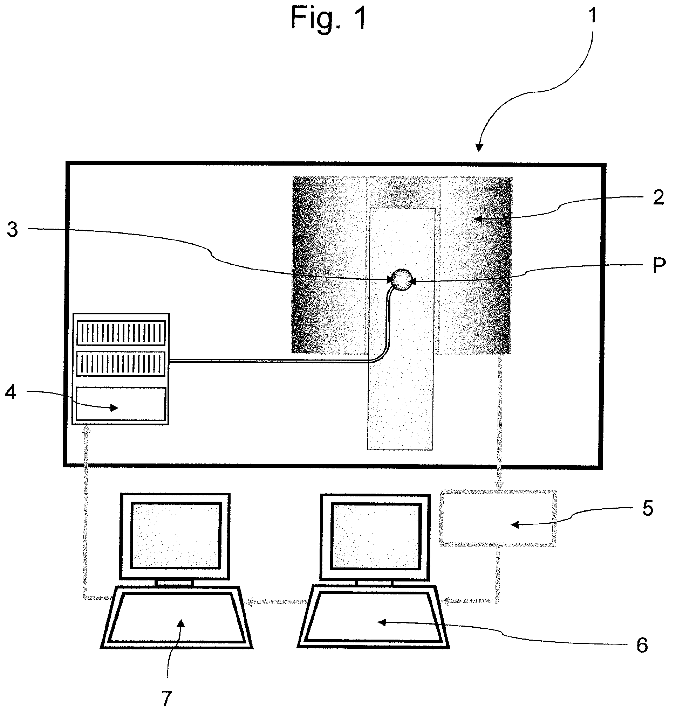

Sur la figure 1 est représenté un dispositif de traitement de tissus biologiques comprenant des moyens de mesure d'une région cible d'un tissu biologique à traiter pour fournir un signal de mesure de la région cible destiné à caractériser la région cible (son mouvement par exemple). On peut par exemple utiliser comme moyens de mesure des moyens d'imagerie IRM 2 destinés à fournir des images de la région cible du tissu biologique à traiter. On utilisera par exemple un appareil d'imagerie IRM de 1,5 Tesla permettant de fournir simultanément des cartes anatomiques et des cartes de température en 3D de la région d'intérêt du patient, avec une résolution spatiale de l'ordre du millimètre, une précision de l'ordre de 0,5°C et une résolution temporelle de l'ordre de la seconde.

Les mesures acquises à l'intérieur de l'aimant sont converties en image par le reconstructeur IRM 5 qui effectue une transformée de Fourier et des filtrages avant d'afficher celle-ci sur la console d'acquisition 6.

Des cartographies de températures incluant la zone à chauffer sont effectuées par l'IRM. Ces données sont transférées en temps réel par une connexion réseau haut débit de la console d'acquisition IRM 6 vers un moyen de contrôle 7 du dispositif se matérialisant par une console de monitorage. Ce moyen de contrôle 7 est notamment dédié au monitorage des cartographies de température et au pilotage du système ultrasonore.

A partir de ces données le moyen de contrôle 7, avec un programme informatique par exemple, évalue selon les cartes anatomiques et thermiques et la position et l'intensité des prochains points de focalisation.

Les coordonnées et la puissance des prochains points focaux sont transmises à des moyens générateurs d'énergie (3,4). Plus précisément, ces données sont transmises à un générateur 4 de signaux 256 voies, via des fibres optiques en quelques millisecondes. Ce générateur 4 produit et amplifie les signaux électriques ultrasonores déphasés de sorte que le transducteur matriciel 3 qui y est connecté émette une onde ultrasonore focalisée au point focal P choisi. L'élévation de température induite à l'intérieur du point de focalisation permet ainsi d'obtenir la dose thermique nécessaire à l'obtention d'une nécrose.

Déplacement électronique du point focal

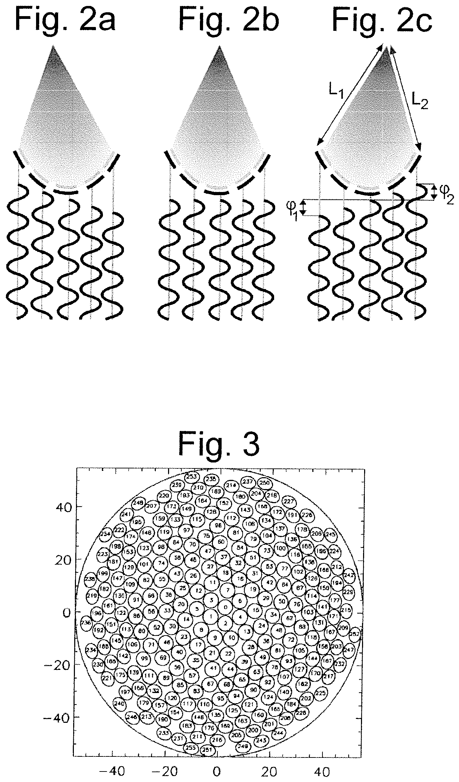

Le principe du déplacement électronique du point focal consiste à ajuster la phase des signaux électriques de façon à créer une interférence constructive à la position voulue des ondes provenant de chacun des éléments. Les figures 2a à 2c montrent un exemple de déphasage des signaux électriques induisant un déplacement électronique du point de focalisation.

Dans le sens de la propagation de l'onde ultrasonore, la phase varie de $2\pi$ pour une longueur d'onde $\lambda$. De ce fait, la phase $\Phi_n$ du signal électrique de l'élément numéro n par rapport à l'élément numéro 0 est calculée selon la loi des phases suivante :

$$\Phi_n = 2\pi \frac{L_n - L_0}{\lambda}$$

(Equation 1)

Dans cette équation la longueur $L_n$ correspond à la distance entre le centre de l'élément n de coordonnées $(x_n, y_n, z_n)$ et le point de focalisation souhaité de coordonnées $(x_F, y_F, z_F)$. Cette longueur se calcule directement par la formule de Pythagore:

$$L_n = \sqrt{(X_n - X_F)^2 + (Y_n - Y_F)^2 + (Z_n - Z_F)^2}$$

(Equation 2)

Cette façon de calculer les déphasages est approximative puisqu'elle suppose l'élément ponctuel, mais elle donne le même résultat que celui obtenu par une analyse plus complète du champ acoustique. Le calcul de la phase des signaux électriques selon l'équation (Equation 1) est très précis et très rapide puisque cela nécessite uniquement le calcul d'une racine carré pour la longueur $L_n$. Durant le contrôle de la température spatiale, le calcul des déphasages étant effectué très fréquemment, cette méthode est toujours celle utilisée.

Comparativement à un déplacement mécanisé du transducteur, le déplacement électronique du point focal est très utile pour contrôler spatialement la température puisqu'il permet de parcourir un grand nombre de points à chauffer par seconde. En plus comme le transducteur reste immobile, ce déplacement du point focal ne crée aucun artefact d'imagerie lié à une modification de la susceptibilité magnétique. Le déplacement électronique du point de focalisation ne possède aucune limitation en vitesse mais elle est en contrepartie soumis à d'autre type de limitation:

- Il n'y pas plus de limitation de vitesse puisque la position du point focal est redéfini à chaque instant par la phase des signaux électriques appliqués sur le transducteur. Seulement le générateur de signaux électriques utilisé nécessite un certain temps de transfert des donnés avant d'effectuer la commutation de toutes les voies à la nouvelle valeur voulue. Par conséquent le temps minimum entre chaque modification de la position du point focal est de 60ms.
- D'autre part l'effet de focalisation ne peut se produire qu'uniquement là où les éléments piezo composites émettent un signal. Cette région est définie par l'effet de diffraction, qui dépend de la taille des éléments, de la fréquence utilisée, de la focal du transducteur et en moindre mesure de sa forme. Pour le transducteur matriciel décrit ci-dessous, le déplacement du point focal est confiné dans un ellipsoïde de dimension 15mm $\times$ 15mm $\times$ 28mm.

Comme il n'y a plus de réelle contrainte sur la vitesse du mouvement, l'asservissement spatial Proportionnel-Intégral-Dérivé (PID) peut être optimisé. On se référera utilement à la demande de brevet française n°99-11418 déposée le 13 septembre 1999 et intitulée « Ensemble de traitement thermique de tissus biologiques et procédé de mise en oeuvre de cet ensemble » pour une description plus détaillée de ce type de système de régulation de la température, que l'on inclut ici par référence. En revanche le principal inconvénient pour les déplacements électroniques est leur faible amplitude de mouvement qui limite le chauffage à des petits volumes.

Description du transducteur matriciel

Pour cette application un transducteur matriciel ultrasonore fonctionnant à 1,5MHz a été développé en collaboration avec la société Imasonic. Ce transducteur de 80mm de focal et de 55mm de rayon d'ouverture forme un angle d'ouverture de 86°. Les éléments choisis pour ce transducteur sont de rayon 2,9mm de façon à avoir une liberté de positionnement du point de focal de -16mm à 13mm le long de l'axe de révolution et de $\pm$7,5mm le long des deux axes orthogonaux. La répartition des éléments à la surface du transducteur est asymétrique compacte de façon à minimiser les lobes secondaire lors du déplacement du point focal.

La figure 3 présente la configuration des éléments retenus pour la fabrication du transducteur. Celle-ci permet de déplacer le point de focalisation sur la plage décrit précédemment avec des lobes secondaires inférieur à 7%.

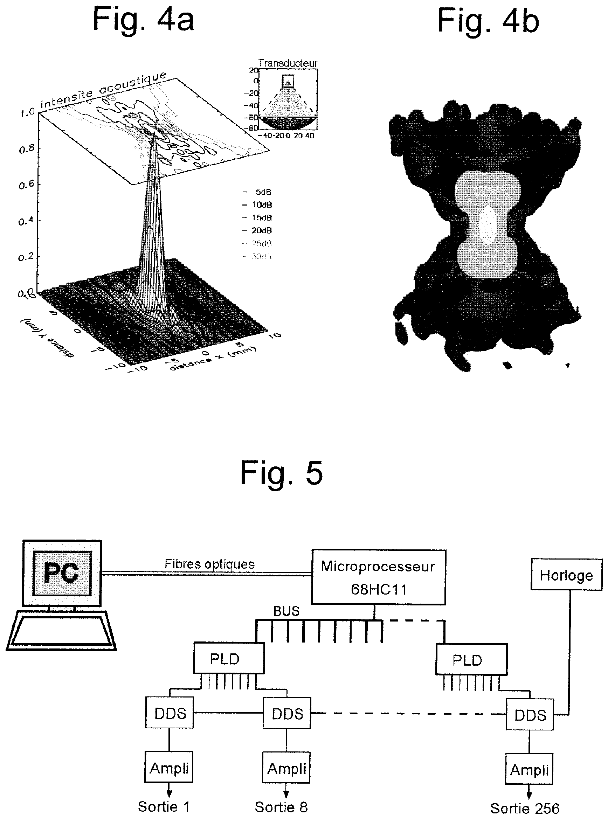

Les figures 4a et 4b représentent une simulation du champ acoustique produit par cette géométrie de transducteur sur une fenêtre de 20$\times$20mm$^2$. Pour ce transducteur d'angle d'ouverture 80°, la taille du point focal est de 0,76x0,76$\times$3,47mm$^3$. Cette géométrie de transducteur permet une utilisation assez générique puisqu'une fenêtre acoustique avec un petit angle d'ouverture suffit.

Pour être parfaitement compatible avec un IRM 1,5T, chacune des pièces utilisées pour la fabrication de ce transducteur ainsi que les câbles et les connecteurs associés sont étudiés pour interférer le moins possible avec le champ magnétique. Pour cela les matériaux choisis, cuivre, étain, plomb, céramique, résine, silicone, plastique ont une susceptibilité magnétique comprise entre celle de l'eau et de l'air. Avec ces matériaux le transducteur n'induit aucune distorsion sur les images IRM. De plus les contours de ce transducteur apparaissent très distinctement sur les images IRM, ce qui permet de repérer facilement sa position et ainsi que la position du point focal produit.

Description du générateur multivoie

L'architecture électronique du générateur de 256 signaux permettant l'utilisation de transducteur matriciel est illustrée à la figure 5.

Une carte MOXA C104H inclus dans le PC permet d'augmenter la vitesse du port série de 115200 Kb/s à 460800 Kb/s. En transmettant uniquement les informations essentielles, fréquence, phases et amplitudes, toutes les informations pour redéfinir les sinusoïdes sont transmises en 22ms.

D'autre part, les fibres optiques, qui en plus de permettre un haut débit sur des grandes distances, réalisent une excellente protection vis à vis des interférences électromagnétique. Celles-ci permettent de faire traverser les informations à travers la Cage de Faraday de l'IRM sans apporter de perturbation provenant de l'extérieur. De cette façon la connexion entre la console de monitorage à l'extérieur de la salle IRM et le générateur à l'intérieur de la salle IRM est assurée sans interférer avec le fonctionnement de l'IRM.

De plus le générateur est développé pour fonctionner à l'intérieur d'une salle IRM. Les cartes ont été conçues pour générer un faible rayonnement électromagnétique. Ainsi son installation nécessite juste l'installation de deux fibres optiques au lieu de 256 boîtiers de filtrages murales pour que chaque câble puisse traverser la cage de Faraday comme cela se faisait auparavant.

Ce générateur est géré par un microprocesseur 68HC11. Il va distribuer les données aux PLD (acronyme de Programmable Logical Device, c'est à dire circuits logiques programmables) sur les 32 cartes qui génèrent 8 sinusoïdes chacune. Les PLD vont ensuite adresser les informations concernant la phase et la fréquence aux DDS (acronyme de Direct Digital Synthesis, c'est à dire générateurs de signaux numériques) et la puissance aux amplificateurs. Les DDS originellement conçus pour la modulation de phase et de fréquence assurent une commutation rapide et précise des signaux. Ceci représente une grande modification par rapport aux autres générateurs de signaux ultrasonores qui avec des lignes à retard ne dépassent pas une précision en phase supérieure à $\pm$5°. Les amplitudes des sinusoïdes sont ensuite ajustées par des amplificateurs à gain variable. L'utilisation d'une horloge commune à tous les DDS assure une utilisation parfaitement synchrone de tous les étages de sorties. Autrement dit, grâce à cette horloge commune la phase de chacune des sinusoïdes est référencée de la même façon.

La définition des signaux se fait par la transmission d'un fichier comportant les données suivant cet ordonnancement :

• 4 octets optionnels pour coder la fréquence commune à toutes les sorties dont la valeur est :

$$F = 24MHz \times \frac{Nb\ cod\acute{e}\ sur\ 32\ bits}{2^{32}}$$

La fréquence est ainsi définie entre 0 et 24Mhz avec une précision de $\pm$3mHz.

• 512 octets définissent sur 2 octets la phase respective de chaque signal suivant la formule :

$$\phi = 360° \times \frac{Nb\ cod\acute{e}\ sur\ 16\ bits}{2^{12}}$$

Ceci permet d'ajuster toutes les phases entre 0 et 360° avec une précision de $\pm$0,04°.

• 256 octets permettent de régler la puissance de sortie de chaque voie selon la relation :

$$P = 4W \times \frac{Nb\ cod\acute{e}\ sur\ 8\ bits}{2^{8}}$$

La puissance de sortie peut ainsi varier jusqu'à 4W avec une précision de $\pm$8mW.

Le protocole de communication est au format X-MODEM en mode connecté half-duplex. Chaque paquet transmis de 128 octets est acquitté par le récepteur qui contrôle la réception par un CRC (acronyme de Cycle Redondance Check) codé sur 1 octet. De cette façon, l'ordinateur peut émettre un fichier définissant la fréquence, les phases ou les amplitudes toutes les 22ms avec un protocole sécurisé.

Ces informations sont ensuite stockées sur une carte mémoires à proximité du microprocesseur 68HC11 puis redistribuées vers chacune des 32 cartes logiques PLD. Lorsque tous les circuits logiques ont reçu leur information, un signal de commutation est envoyé pour toutes les cartes simultanément. A partir de cet instant chaque circuit logique modifie la fréquence, la phase et l'amplitude de toutes les sinusoïdes en même temps. Ceci permet d'éviter les états transitoires où le faisceau ultrasonore est partiellement défocalisé et donc néfaste à la sécurité du patient.

Cependant lié à des limitations imposées par le programme implémenté dans le microprocesseur, le temps minimal de modification de l'état de toutes les sorties et limité à 60ms.

En résumé, ce générateur est conçu de façon à associer :

• Rapidité de redéfinition des signaux
• Changement simultané des signaux
• Faible rayonnement électromagnétique
• Précision de la définition des sinusoïdes de sorties
• Un échange de donnés sécurisés
• Portabilité

Exemple de déplacement électronique du point focal

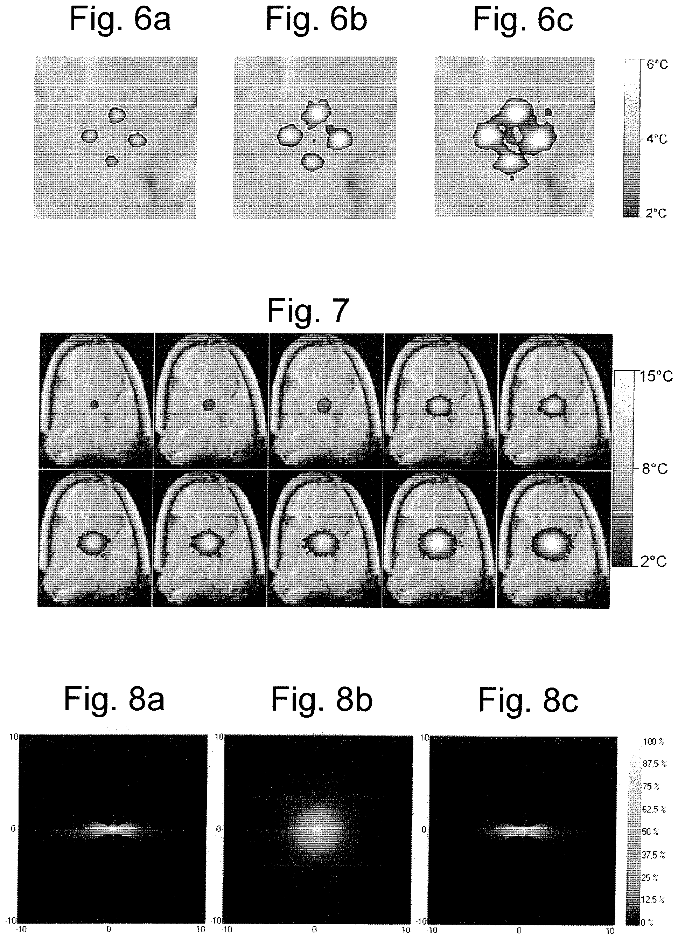

Pour tester le principe de déplacement électronique du point focal et le bon fonctionnement du transducteur matriciel, le point de focalisation a été déplacé en 4 points selon un carré de 8mm de coté. Cette trajectoire se répète de façon cyclique toutes les 0,5s en changeant la position du point de focalisation toutes les 125ms avec une puissance constante de 200W électrique. En effectuant simultanément 5 coupes de 4mm (temps d'écho 18ms) toutes les 3,9s, le chauffage observé aux figures 6a à 6b semble provenir de 4 points de focalisation simultanément.

Cette méthode de déplacement du point focal est très rapide puisqu'il n'y a plus de vitesse maximale de déplacement mais juste un temps minimal de commutation électronique des signaux de 60ms avec le générateur utilisé. Cette technique est aussi très précise. La précision de définition de phase des signaux produits est une fraction de $2^{12}$ d'une période correspondant à une longueur d'onde de 1 mm. De plus les erreurs de phase entre les 256 signaux se moyennent entre eux. La précision de positionnement théorique du point focal est de $1mm/(256 \times 2^{12})$=1nm. Ce résultat reste toutefois difficile à vérifier même par des simulations qui nécessiteraient un très petit maillage et une très grande précision numérique.

Le type de trajectoire réalisable peut être choisi de façon quelconque à condition que chaque point ne s'écarte pas de plus de 7,4mm de sa position central. La figure 7 montre toutes les cartographies thermiques obtenues durant deux chauffages spiralés successifs. Chaque spirale de diamètre 10mm est constituée de 96 points et dure 20s. La séquence utilisée est la même que précédemment à raison d'une acquisition toutes les 3,9s.

La puissance appliquée en chacun des points est constante de 50W électrique compensé en intensité en fonction de l'amplitude du déplacement. De cette façon le chauffage obtenu est homogène tant que le tissu ne présente pas d'hétérogénéité, ce qui semble être le cas sur cette expérience. Le cas échant la puissance ou la densité de points peut être contrôlée par l'algorithme d'asservissement de la température 2D présenté plus loin. Cependant le point focal pouvant être déplacé électroniquement dans toutes les directions de l'espace, il est préférable d'effectuer un asservissement tridimensionnel (3D) de la température.

## Contrôle de la température volumique

Répartition énergétique 3D selon l'algorithme PID spatial

Le contrôle spatial de la température est fondé sur une extension de l'équation différentielle d'automatise PID à chacun des points de l'espace :

$$\frac{\partial \xi}{\partial t}(\vec{r}) + q\,\xi(\vec{r}) + \frac{q^2}{4}\int_0^t \xi(\vec{r}) = 0$$

(Equation 3)

Dans cette équation la variable spatiale et temporelles $\xi$ est l'écart entre la température de consigne $T_C$ et la température mesurée $T$ au cours du temps t en chacun des points $\vec{r}$ l'espace.

$$\xi_{(\vec{r},t)} = T_{C\,(\vec{r},t)} - T_{(\vec{r},t)}$$

(Equation 4)

Le principe du contrôle spatial et temporel de la température traite chacun des points de l'espace individuellement. Ainsi la température de consigne de chacun des points chauffés peut-être définie indépendamment les uns des autres. Cependant la convergence à la température voulue n'est pas toujours garantie car il est difficile de chauffer un point isolement. Au voisinage du point focal, surtout le long de l'axe de propagation de l'onde, il subsiste toujours une montée en température. D'autre part, l'effet de diffusion thermique tend à propager le chauffage d'un point au point voisin. Pour ces raisons le contrôle de la température peut se faire à condition que la résolution spatiale de la consigne de température ne dépasse pas la résolution spatiale du point focal et que le gradient de température entre deux points voisins ne soit pas trop élevé par rapport au coefficient de diffusion. L'équation de transfert thermique utilisée pour anticiper le comportement du tissu et l'interaction des différents points chauffés est la suivante:

$$\frac{\partial T}{\partial t}(\vec{r}) = D \cdot \nabla^2 T(\vec{r}) + \alpha \cdot P(\vec{r})$$

(Equation 5)

Ainsi l'équation différentielle PID est équilibrée lorsque la puissance appliquée est choisi de sorte que:

$$P(\vec{r}) = \frac{1}{\alpha}\left[\frac{\partial T_C}{\partial t}(\vec{r}) - D \cdot \nabla^2 T(\vec{r}) + q\left(T_C(\vec{r}) - T(\vec{r})\right) + \frac{q^2}{4}\int_t \left(T_C(\vec{r}) - T(\vec{r})\right)\right]$$

(Equation 6)

Il est très difficile d'appliquer une répartition spatiale de la puissance, c'est-à-dire de chauffer plusieurs points simultanément. Cela peut se faire en utilisant un transducteur matriciel qui focalise simultanément en plusieurs points. Cette utilisation des transducteurs matriciels est cependant limitée et très dangereuse car elle induit de nombreux lobes secondaires indésirables. Un résultat de meilleure qualité est obtenu plus simplement en focalisant successivement en chacun des points.

Quelque soit la nature du déplacement du point focal, électronique ou mécanique, il est nécessaire de prendre en compte l'énergie à déposer en chacun des points plutôt que la répartition spatiale de la puissance à appliquer :

$$E(\vec{r}) = \frac{t_A}{\alpha}\left[\frac{\partial T_C}{\partial t}(\vec{r}) - D \cdot \nabla^2 T(\vec{r}) + q\left(T_C(\vec{r}) - T(\vec{r})\right) + \frac{q^2}{4}\int_t \left(T_C(\vec{r}) - T(\vec{r})\right)\right]$$

(Equation 7)

L'intervalle de temps $t_A$ est la durée nécessaire pour parcourir l'ensemble des points à chauffer. Cette période de temps définit aussi la fréquence à laquelle l'asservissement PID est calculé. Pour garantir la stabilité de la contre réaction, le temps de réponse du système $t_R$ (égal à 2/q) doit être supérieur au temps d'asservissement $t_A$. En général le temps de réponse est choisi égal à 3 fois le temps d'asservissement.

Pour l'implémentation de l'asservissement PID de régulation, le terme proportionnel intégral et dérivé se calcule de la même façon que pour un asservissement ponctuel. De même l'anticipation du comportement peut se calculer par FFT (acronyme de Fast Fourier Transform, c'est à dire transformation de Fourier rapide) ou de façon discrète. Comme l'asservissement PID spatial est basé sur les mêmes équations que l'asservissement PID ponctuel, il offre la même stabilité vis à vis des paramètres tissulaires utilisés.

Pour mieux contrôler le volume chauffé il est préférable d'effectuer un contrôle spatial 3D de la température en utilisant l'équation (Equation 7) sur tout le volume. Cependant cela nécessite de pouvoir déposer la répartition spatiale d'énergie 3D ainsi calculée. Ceci est assez complexe compte tenu du grand nombre de voxel contenu dans le volume et surtout de l'effet de chevauchement décrit précédemment.

Méthode de dépôt énergétique 3D

L'équation générique (Equation 7) permet de calculer dans tous les cas l'énergie requise pour obtenir la température voulue en trois dimensions. Bien que le transducteur matriciel associé à un générateur de signaux multivoie soit un outil très rapide, il n'est pas toujours simple de produire cette énergie.

Compte tenu des limitations matérielles, certaines répartitions spatiales énergétiques ne sont pas réalisables. Par exemple, le calcul de l'équation PID (Equation 7) en un point largement plus chaud que sa température de consigne aboutit en général à une valeur énergétique négative. Bien qu'il existe des outils cryothérapeutiques, les transducteurs ultrasonores ne permettent pas d'extraire une quantité d'énergie. Par conséquent, l'asservissement PID n'est plus faisable en ce point. Il ne reste alors plus qu'à attendre que l'effet de diffusion ou de perfusion refroidisse suffisamment le tissu.

De même si la répartition spatiale énergétique requise est un Dirac de $1 \times 1 \times 1 mm^3$, elle n'est pas réalisable avec un point de focalisation de $0,76 \times 0,76 x 3,47 mm^3$ trois fois plus allongé. Il est donc nécessaire de prendre en compte la répartition spatiale de l'énergie $E^{1pt}$ induite par un point de focalisation. Si le déplacement du point focal peut être assimilé à une translation, l'énergie déposée par une trajectoire $E^{Traj}$ se calcule rapidement dans l'espace de Fourier par la formule suivante:

$$\widetilde{E}^{Traj} = \widetilde{\Gamma} \times \widetilde{E}^{1pt}$$

(Equation 8)

Dans cette expression la fonction trajectoire Γ est la répartition spatiale de la densité de points. Jusqu'à présent pour le contrôle de la température en 2D, l'énergie produite par une trajectoire était assimilée à la densité de points puisque dans le plan coronal le point focal correspondait à un voxel. Cette règle est vérifiée tant que la largeur du voxel acquis par l'IRM dans le plan coronal ne dépasse pas 0,76mm. Pour une étude tridimensionnelle, cette approximation n'est jamais vérifiée à cause de l'effet de chevauchement du cône ultrasonore de propagation. Cet effet peut induire des chauffages de plusieurs centimètres le long de l'axe de propagation comme le montre l'exemple des trajectoires spiralées. L'ensemble des répartitions énergétiques produites est par conséquent aussi limité par ce phénomène de chevauchement qui dépend de la répartition spatiale du champ acoustique. D'après l'équation (Equation 8), cet ensemble est directement lié à la transformée de la forme du point focal représenté aux figures 8a à 8c.

Puisque la transformé de $E^{1pt}$ est nulle pour la majorité des points où $k_Y$ est non nulle, il en va de même pour la transformé de $E^{Traj}$. En revanche $E^{1pt}$ couvre complètement le plan $k_X k_Z$. En d'autre terme l'ensemble des répartitions énergétiques réalisables ne peut comporter de détail dans la direction Y mais peut en comporter dans les directions X et Z. De façon générale dans l'espace de Fourier, les répartitions énergétiques réalisables sont nulles là où la transformé de $E^{1pt}$ est nulle. De plus il faut qu'il existe une fonction trajectoire positive vérifiant l'équation (Equation 8) puisque la densité de points ne peut être négative.

Pour effectuer un contrôle de la température il faut effectuer le travail inverse à celui effectué pour la simulation du champ acoustique. Au lieu de calculer la répartition énergétique à partir de la trajectoire, il faut déduire la trajectoire d'une répartition énergétique. Compte tenu des contraintes énoncées précédemment, ce problème peut se résoudre par une déconvolution comme le décrit l'équation suivante:

$$\widetilde{\Gamma} = \widetilde{E}^{Traj} / \widetilde{E}^{1pt} \quad \text{si} \quad \widetilde{E}^{1pt} \neq 0$$
$$\widetilde{\Gamma} = 0 \quad \text{si} \quad \widetilde{E}^{1pt} = 0$$

(Equation 9)

Dans l'espace de Fourier si $E^{Traj}$ est non nulle alors Γ correspond au rapport de $E^{Traj}$ par $E^{1pt}$ et si $E^{Traj}$ est nulle Γ est choisie égale à zéro pour minimiser l'énergie déposée par cette trajectoire. Ensuite dans l'espace réel, seules les valeurs positives de la fonction trajectoire sont conservées.

Malheureusement, cette solution minimisant la différence entre l'énergie requise et l'énergie produite sur toute la fenêtre d'observation, tend à diminuer l'énergie sur le volume cible pour minimiser l'énergie déposée à l'extérieur. Selon la taille de la fenêtre d'observation et donc le nombre de voxel en dehors du volume cible, la sous évaluation de l'énergie à déposer est plus ou moins importante. De plus, le produit de convolution par une fonction positive $E^{1pt}$ est un filtre passe bas. La déconvolution par $E^{1pt}$ produit l'effet inverse ce qui rehausse les contours et amplifie le bruit. La fonction trajectoire obtenue par la méthode de la déconvolution étant dépendante de la taille de la fenêtre d'observation et présentant un rapport signal sur bruit inférieur à celui de la répartition énergétique calculée, il est nécessaire d'utiliser une autre solution pour conserver la stabilité de l'asservissement PID.

Algorithme de détection du maximum

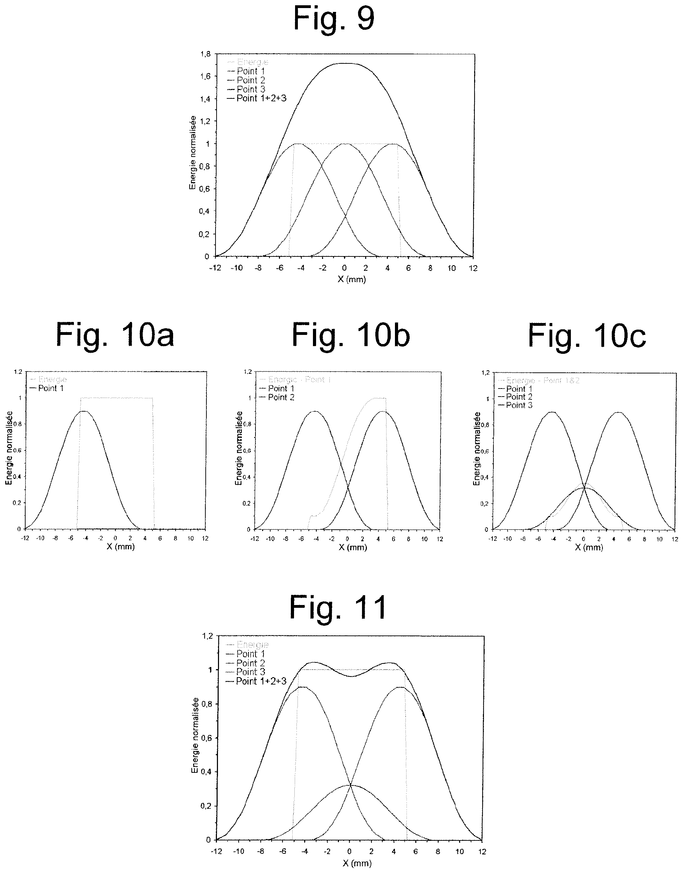

La solution triviale utilisée pour le contrôle de température 2D consistant à choisir la fonction trajectoire égale à la répartition spatiale d'énergie requise est efficace tant que l'effet de chevauchement est négligeable. Pour illustrer cela, la figure 9 présente trois formes de points focaux additionnées dans le but de réaliser une répartition spatiale carrée.

Sur ce graphique la courbe rouge représente l'énergie requise et la courbe noire présente l'énergie produite par les points de focalisation translatés. Les pieds de chaque point focal ou leurs lobes secondaires à plus longue distance s'additionnent aux autres points de focalisation qui en leur centre atteignent déjà la valeur de l'énergie requise. Par conséquent l'énergie produite sur cet exemple dépasse de 70% le niveau souhaité. La forme du point focal ici utilisé correspond à l'intensité relevée le long de l'axe de propagation de l'onde élargie d'un facteur 2 pour accentuer le chevauchement. En pratique la superposition se faisant en trois dimensions, ce dépassement atteint des valeurs bien plus grandes.

Une solution moins radicale consiste à déduire chaque point par itération en observant l'influence des autres points. Le but de cet algorithme est de trouver la trajectoire de plus faible énergie pour atteindre le niveau d'énergie requis en tous les points de l'espace.

Le principe de la focalisation est de concentrer toute l'énergie émise en un point. De ce fait pour une énergie requise ponctuelle, le meilleur rendement est obtenu en focalisant en ce point. De même, dans le cas d'une répartition spatiale quelconque de l'énergie requise, il est optimal de focaliser au point où l'énergie requise est maximale. Cependant l'énergie de la focalisation appliquée en ce point dépend des points de focalisation réalisés autour de celui-ci. Pour ne pas reproduire l'erreur illustrée en figure 9, l'énergie appliquée en ce point doit être inférieure au niveau énergétique requis. De façon arbitraire l'énergie à appliquer en ce point est alors choisie égal à un pourcentage R de la valeur de l'énergie requise. Une fois ce point de focalisation à appliquer pris en compte, l'algorithme est itéré en cherchant le point correspondant au maximum de la répartition d'énergie requise retranchée de la ou des répartitions d'énergie émanant du ou des points de focalisation précédemment déduits. Les figures 10a à 10c représentent les trois premières itérations de cet algorithme appliqué sur une fonction carré, avec un pourcentage R de 90%.

En notant $E^i(\vec{r})$ la différence entre l'énergie requise et l'énergie produite par les $i$ points de focalisations antérieurement déduits et $\Gamma^i(\vec{r})$ la fonction trajectoire correspondante, cet algorithme s'écrit mathématiquement sous la forme de la suite suivante:

$$E^0(\vec{r}) = E^{REQUIS}(\vec{r}) \quad \text{et} \quad \Gamma^0(\vec{r}) = 0$$

$$\left| \begin{array}{l} \text{soit } \vec{r}_n \text{ tel que } E^n(\vec{r}_n) = \max(E^n(\vec{r})) \\ E^{n+1}(\vec{r}) = E^n(\vec{r}) - R \cdot E^n(\vec{r}_n) \cdot E^{1pt}(\vec{r} - \vec{r}_n) \\ \Gamma^{n+1}(\vec{r}) = \Gamma^n(\vec{r}) + R \cdot E^n(\vec{r}_n) \cdot \delta(\vec{r} - \vec{r}_n) \end{array} \right.$$

(Equation 10)

Dans cette équation, la fonction de Dirac $\delta$ vaut 0 partout excepté à l'origine où elle vaut 1. Le contrôle de la température étant effectué sur un nombre fini de voxel N, après N itérations le maximum de l'énergie restante $E^n$ décroît d'au moins un facteur 1-R:

$$\max(E^{n+N}(\vec{r})) \leq (1-R) \cdot \max(E^n(\vec{r}))$$

(Equation 11)

Cette inégalité certifie une convergence géométrique telle que le maximum de l'énergie restante converge vers 0. L'énergie du point focal à appliquer au maximum est choisie égale à un pourcentage de l'énergie restante au lieu d'un pas constant pour obtenir cette convergence géométrique plutôt qu'une convergence arithmétique. D'autre part la convergence de la suite de fonction trajectoire $\Gamma^n$ est garantie puisque chacun de ses points représente une suite croissante bornée par la fonction triviale énoncé figure 9.

À la convergence, la fonction trajectoire $\Gamma$ obtenue correspond à celle de plus petite énergie atteignant en chacun des points l'énergie requise. Si la répartition énergétique requise ne fait pas partie de l'ensemble des fonctions réalisables (comme la fonction carrée) l'énergie produite par cette trajectoire dépasse en certains points le niveau énergétique voulu (sur les bords extérieurs du carré). Mais au moins la nécrose est obtenue sur tous les voxels voulus à moindre énergie.

La figure 11 montre l'énergie produite par les trois points de focalisation établit à la figure 10. La convergence n'est pas atteinte puisque le point central n'atteint pas l'énergie requise. L'itération suivante consisterait à augmenter la valeur du point central. Dans tous les cas la convergence n'est jamais atteinte puisque à chaque étape il reste toujours un pourcentage 1-R du maximum. Pour cette raison il est indispensable d'utiliser un critère d'arrêt. Celui retenu est la comparaison du dernier maximum avec un pourcentage du maximum initial, ce qui permet de définir la précision de l'énergie produite obtenue.

D'autre part le facteur R définit la vitesse de convergence de l'algorithme selon l'équation (Equation 11). Plus R est proche de 1 et plus la convergence est rapide, mais plus il y a de risque d'obtenir une valeur trop élevée pour l'estimation de l'énergie à déposer au point maximum détecté. Pour éviter un tel dépassement inutile de l'énergie produite, 1-R peut être choisi supérieur ou égal à la somme des pieds des points de focalisations connexes normalisés à un. Comme ce résultat dépend de l'espacement des points de focalisation et donc de la résolution de l'imagerie IRM, il est plus simple de choisi R relativement faible. Ce choix n'est pas très contraignant puisque même avec un facteur de convergence de 10% pour une précision de 2% le temps de calcul n'excède pas 10ms pour processeur 1GHz. Ceci nécessite toutefois d'optimiser le code en cherchant le maximum et en calculant l'énergie restante uniquement sur les N voxels où l'asservissement est effectué. Dans le cas contraire le code est ralenti d'un facteur 100 ou plus selon la taille de la fenêtre d'observation.

La fonction trajectoire obtenue est identique quel que soit le facteur R choisi à condition qu'il ne dépasse pas une certaine valeur. La seule ambiguïté réside dans le choix du maximum en cas d'égalité, ce qui est le cas pour la première itération appliqué à une énergie requise uniforme comme le carré précédemment présenté. En pratique ce cas singulier ne se produit pas fréquemment et le choix d'un maximum plutôt qu'un autre n'a pas de réelle conséquence puisque les deux nécessitent un dépôt énergétique. La solution retenue est le premier maximum rencontré car les régions situées à la périphérie du volume chauffé nécessitent une densité de points plus élevée.

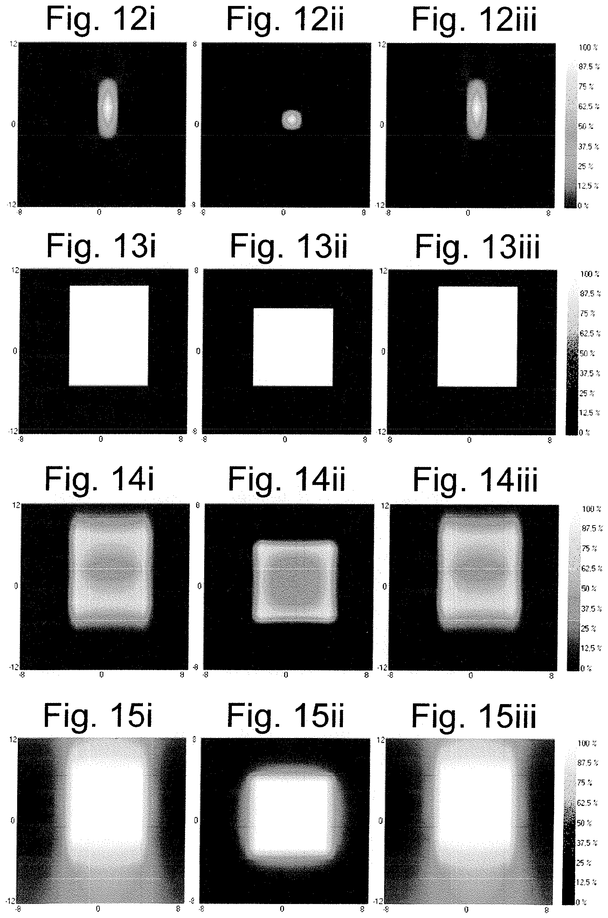

Les figures 12 à 15 illustrent la mise en application de cet algorithme de détection du maximum sur un volume 3D cubique de $7\times7\times9mm^3$ avec un facteur R de 10% et une précision de 5%. Similairement à l'IRM la résolution utilisée est de 6 coupes composées de voxel de $1\times1\times4mm^3$. La densité de points obtenue se situe principalement dans les coins du cubes puis sur les arrêtes et préférentiellement sur la face supérieure et inférieure. En bref c'est une forme creuse. En général, quelque soit la forme de l'énergie requise, la majeure partie de la densité de points se situe à sa périphérie.

Pour vérifier l'exactitude du résultat, l'énergie produite calculée selon l'équation (Equation 8) avec cette densité de points est aussi reportée aux figures 15i, 15ii, et 15iii. Comme prévu, celle-ci correspond à l'énergie requise à 5% près sur la totalité du cube. En revanche une énergie est inévitablement produite à l'extérieur du cube. Celle-ci est surtout située le long de l'axe de Y, là ou se chevauche les cônes de propagation ultrasonore.

L'algorithme de détection du maximum permet de définir la densité de points précisément, rapidement et de façon stable. Comme les hautes fréquences du bruit présentent des gradients élevés non réalisables (surtout selon l'axe Y), le signal sur bruit de la fonction trajectoire obtenue est supérieur ou égal à celui de la répartition énergétique requise. De plus, dans le cas où cette répartition n'est pas réalisable, l'énergie produite excède en certains points l'énergie requise de façon à atteindre la température voulue en tous les points avec une énergie minimum. Tous ces avantages correspondent parfaitement au besoin thérapeutique, le seul inconvénient réside dans le dépassement moyen de l'énergie requise qui augmente avec le niveau de bruit et le nombre de voxels contrôlés.

Extraction d'une trajectoire

Une fois la répartition spatiale de la densité de points calculée à partir de l'algorithme de détection du maximum, il faut en extraire une trajectoire qui prend en compte toutes les limitations matérielles énoncées précédemment.

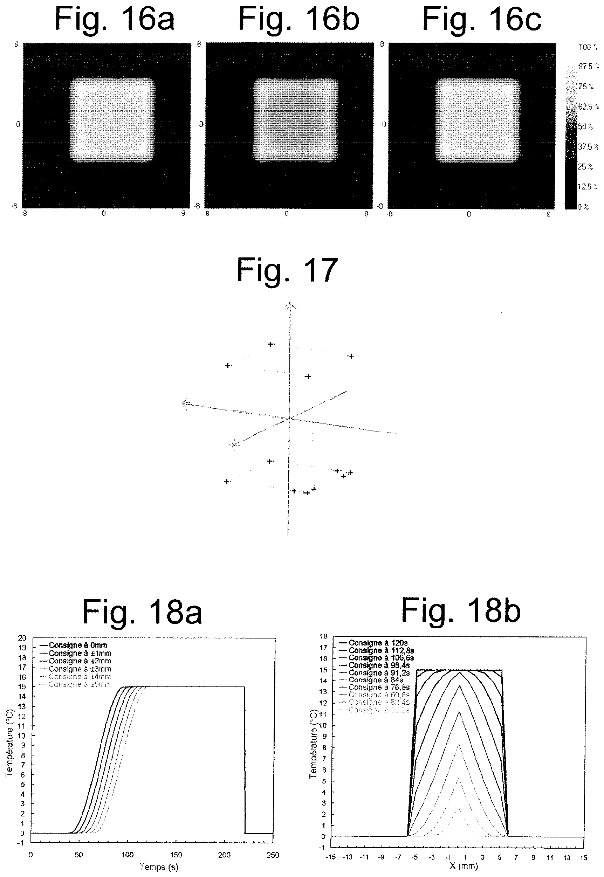

La fonction trajectoire obtenue précédemment (voir les figures 16a à 16c) est composée de 3 coupes sur lesquels un carré de $7\times7$ voxels est non nul. L'idéal serait de réaliser une trajectoire de 147 points de façon à produire l'énergie requise à 5% près. Cependant la durée de la trajectoire est choisie égale à la durée d'une dynamique pour pouvoir effectuer la contre réaction le plus fréquemment possible. Ainsi le temps d'asservissement est choisi égal à la durée d'acquisition d'un dynamique soit ici 2,4s. Compte tenu du fait que le temps minimal de commutation du générateur est de 60ms, cela ne permet de réaliser qu'au maximum 40 points durant cet intervalle de temps. De plus il faut utiliser une trajectoire de durée plus courte que le temps d'acquisition d'une dynamique puisque le temps de transmission et de traitement d'une image n'est pas parfaitement régulier. Il est aussi très utile de garder une marge de flexibilité d'un facteur 2 quant à la durée des points. Cette liberté permet de réaliser les points nécessitant des niveaux d'énergie très élevé avec une longue durée et une puissance modérée plutôt qu'un chauffage court et avec une puissance très élevé. En effet les forts niveaux de puissance acoustique même très brefs suffisent à déclencher un effet de cavitation qui modifie le comportement du tissu, ce qui n'est pas l'effet recherché ici. Au final, seule une trajectoire durant 2,2s avec 12 points, comme celle représentée à la figure 17, est retenue parmi les 147 points de la fonction trajectoire initiale.

Les points conservés sont ceux de plus haut niveaux énergétique, les autres points sont en général réalisés à la trajectoire suivante si le calcul de la contre réaction le juge nécessaire. L'ordonnance temporelle de ces points parmi la trajectoire ne semble pas avoir d'effet significatif sur le chauffage induit compte tenu des faibles durées mises en jeux.

Une fois la position des points et leur niveau énergétique définis, il est possible de réaliser cette trajectoire en ajustant la durée ou la puissance de chacun de ses points. Lorsque ce choix est possible, il est préférable de modifier la durée plutôt que la puissance. Il est bien plus prudent d'utiliser des émissions de longue durée que de forte puissance, aussi bien pour l'usure du matériel que pour la sécurité du patient. Evidement la durée d'un point ne doit pas dépasser 2,2s puisque la somme de tous les points doit être égale à la durée de la trajectoire choisie. De même une limitation minimale est imposée pour chacun des points à 80ms puisque le temps de commutation de tous les signaux du générateur est limité. Concernant les puissances utilisées elles sont ici limitées au maximum à 200W électrique mais elle ne possède pas de limite inférieure. Evidement lorsqu'une puissance nulle est associée à un des points de la trajectoire celui ci est automatiquement supprimé de la liste.

En bref toutes ces contraintes, parfois contradictoires, nécessitent l'utilisation d'un algorithme pour définir la durée et la puissance de chacun des points. Cet algorithme est initialisé en associant une puissance identique à chaque point mais avec une durée variable calculée de façon à avoir la répartition énergétique souhaitée. La constante de puissance initialement choisie n'a aucune importance puisqu'elle est modifiée par la suite.

La première étape de la boucle d'itération consiste à modifier la durée de tous les points par un même facteur de façon à obtenir la durée de la trajectoire voulue. La puissance de chacun des points est divisée par ce même facteur pour conserver l'énergie voulue en chacun d'eux.

La seconde étape à pour but d'imposer un temps minimum à tous les points de trop courte durée. Pour les points dont la durée a été rallongée, la puissance est diminuée pour conserver l'énergie initialement définie.

La troisième et dernière étape est de tronquer la puissance de tous les points excédant la puissance maximum choisie. Pour préserver l'énergie prédéfinie, la durée des points modifiés est alors augmentée.

Ces deux dernières étapes ayant augmenté la durée totale de la trajectoire, l'algorithme réitère le calcul à la première étape une dizaine de fois. Cet algorithme s'exécute très rapidement (en un temps inférieur à la milliseconde) puisque le nombre de points traités est faible (12 points). Toutefois dans certains cas, il n'existe pas de trajectoire respectant ces limitations avec l'énergie voulue. Par exemple si l'énergie totale voulue est supérieure au produit de la puissance maximum par la durée de la trajectoire, ou si le nombre de points multiplié par le temps minimum dépasse la durée de la trajectoire. Mais en sortant de la boucle d'itération, les limitations sur la puissance maximum et le temps minimum sont toujours vérifiées. Seule la durée de la trajectoire peut-être plus longue que prévue. Dans ce cas, les derniers points ne sont pas réalisés. Comme les autres points de la fonction trajectoire initiale, ces points sont réalisés durant la dynamique suivante.

Le passage de l'énergie requise à la fonction densité de points est limité par la forme du point focal et donc du transducteur. Ensuite la conversion de la fonction trajectoire en une trajectoire discrétisé est plutôt limitée par le générateur de signaux utilisé.

Décalage temporel de la consigne de température

Les méthodes décrites jusqu'à présent permettent de calculer la trajectoire pour atteindre la température voulue. Il suffit alors de choisir convenablement la température de consigne pour effectuer le chauffage voulu. Le plus souvent celle-ci est définit temporelle par une température cible de 12°C à 15°C durant quelques minutes de façon obtenir la dose thermique nécessaire à l'ablation des tumeurs. Cette consigne peut être choisie beaucoup plus faible pour une utilisation non destructive comme l'expression génique ou le dépôt local de médicament. La phase transitoire de la montée en température est obtenue par une demi-période de sinusoïde. Ceci permet de ne pas créer de discontinuité de la courbe de consigne et de sa dérivée, ce qui est plutôt favorable à la stabilité de l'asservissement.

Concernant la répartition spatiale de la consigne de température, elle est choisie en général uniforme sur toute la tumeur et nulle en dehors dans le but d'obtenir un traitement homogène sur la région cible tout en protégeant au mieux les tissus voisins. Ainsi la consigne de température est une fonction carrée dont l'amplitude varie selon la consigne temporelle définie préalablement.

Toutefois la montée en température de tout un volume nécessite souvent une quantité d'énergie importante, ce qui peut durer une à deux minutes compte tenu des limitations matérielles et de la taille du volume. Cependant durant cet intervalle de temps le chauffage se propage vers l'extérieur par effet de diffusion. Une méthode pour limiter cet effet consiste à modifier la réparation de la consigne de façon à terminer la montée en température par les points situés à périphérie du volume comme pour une trajectoire spiralée. Pour cela la même consigne temporelle est utilisée pour chacun des points avec un léger décalage proportionnel à sa distance par rapport au point central. De cette façon le point central est chauffé en premier. Ce chauffage est ensuite propagé aux points voisins avec une vitesse définie par l'opérateur. Les figues 18a et 18b montrent un exemple de consigne utilisé par la suite pour chauffer un segment de 11 voxels (de-5mm à +5mm) en décalant cette consigne de 5s pour chaque millimètre.

Par conséquent les consignes temporelles sont translatées les unes des autres à l'exception de la fin du traitement qui est synchronisé pour l'ensemble des points. En effet, les points centraux ne peuvent pas refroidir avant les points périphériques. La répartition spatiale de la consigne de température prend une forme similaire à la consigne temporelle sur un intervalle de 25s. Si la montée en température est linéaire au cours du temps, la consigne spatiale forme une droite et de même si elle s'incurve. Par effet de symétrie par rapport au point central, la consigne spatiale de température forme initialement un triangle d'amplitude et de largeur croissantes. Une fois la largeur et l'amplitude voulues atteintes cette consigne spatiale s'arrondie pour former progressivement la fonction carré permettant par la suite le traitement homogène.

**Résultats expérimentaux**

Pour évaluer l'efficacité de cette méthode de contrôle de la température plusieurs asservissements ont été effectués avec le transducteur matriciel et le générateur 256 voies présenté précédemment. La séquence d'acquisition utilisée est toujours la même avec 6 coupes de $128 \times 128$ voxels de dimension $1 \times 1 \times 4\text{mm}^3$. La séquence en écho de gradient utilisée a un temps d'écho de 18ms et temps de répétition de 300ms. De plus des techniques de traitement l'image connues ont été systématiquement appliquées aux résultats présentés ci dessous.

## Contrôle de la température linéaire

Le premier contrôle spatial effectué couvre un segment de 11 mm aligné le long de l'axe X. La consigne de température utilisée est un chauffage homogène de 15°C durant 120s pour obtenir 3,5 fois la dose thermique. La montée en température est dans un premier temps effectuée de façon synchronisée pour les 11 voxels contrôlés.

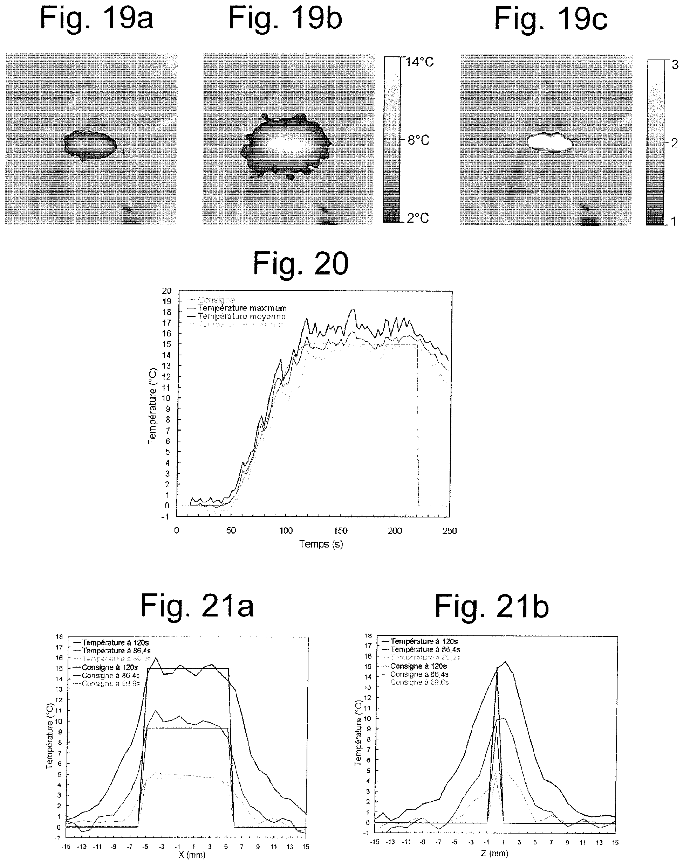

Les figures 19a et 19b représentent deux cartographies de température acquises au milieu et à la fin de la montée en température. La forme chauffée correspond exactement à celle voulue, soit un segment de largeur 11 mm de température homogène. Comme toujours la diffusion thermique propage la température au tissu voisin ce qui élargie légèrement la nécrose obtenue qui comme le montre la carte de dose thermique figure 19c à la fin du chauffage mesure $12 \times 4mm^2$.

Pour une étude plus statistique que visuelle, la figure 20 représente la température minimum, moyenne et maximum pour les 11 voxels où l'asservissement PID est effectué. L'ensemble de ces points suit la consigne de température à 0,6°C près en moyenne. L'écart entre le point le minimum et le maximum est d'environ 2°C durant tout le chauffage. Par conséquent tous les points contrôlés induisent la dose thermique voulue selon la consigne choisie.

Les figures 21a et 21b représentent la répartition de la température au début, au milieu et à la fin de la montée en température le long des axes X et Z. L'élévation de température produite par les multiples points de focalisation forme un chauffage homogène dont l'amplitude varie selon la consigne spatiale et temporelle préalablement choisie. Seul l'effet de la diffusion thermique qui s'oppose constamment à l'établissement de gradient de température, empêche la réalisation d'une fonction carrée. La diffusion thermique élargie dans toutes les directions de l'espace la zone chauffée.

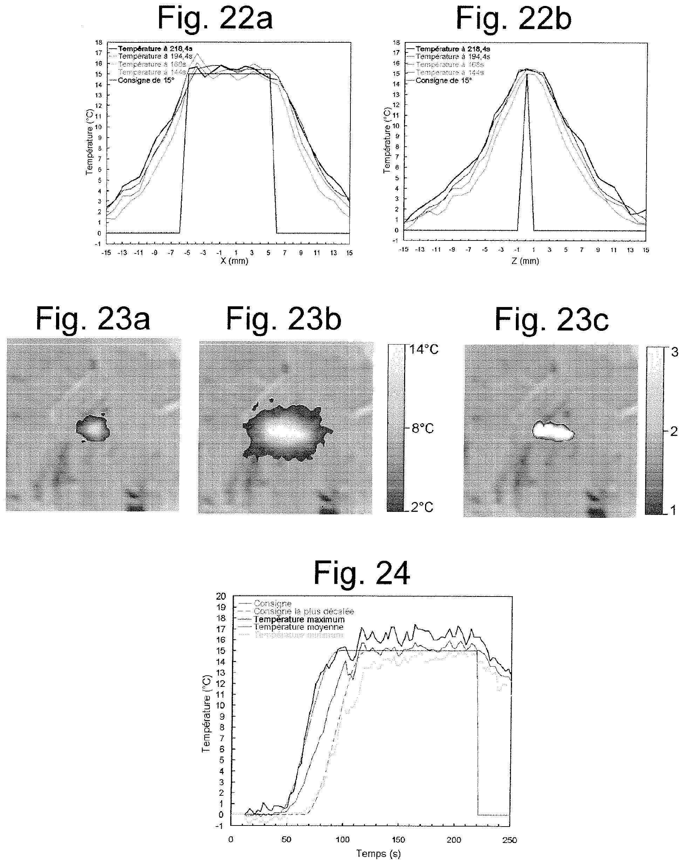

Comme le montre les figures 22a et 22b, une fois la consigne de température de 15°C atteinte celle-ci est maintenue précisément jusqu'à la fin du chauffage. La seule différence entre les différentes cartographies thermiques est l'élargissement progressif de la zone chauffée par diffusion thermique.

## Contrôle de la température linéaire décalé

L'expérience précédente a été reproduite en remplaçant la consigne de température par celle représentée aux figures 18a et 18b dont la montée en température est légèrement différée pour les points éloignés du centre. Le but de cette modification est de diminuer l'étalement de la température. Pour être comparable au chauffage précédent cette nouvelle consigne a été choisie comme précédemment avec une montée en température durant 80s pour l'ensemble des points. De même la température de consigne maximale est de 15°C durant 100s pour produire la même dose thermique sur les points périphériques. Les points centraux sont légèrement plus nécrosés puisqu'ils atteignent le maximum de 15°C légèrement plus tôt.

Les figures 23a et 23b montrent les deux cartes thermiques obtenues au milieu et à la fin de la consigne de température, c'est à dire aux dynamiques 32 et 48 respectivement. Comme prévu le point central est chauffé en premier puis les points voisins suivent pour obtenir finalement le chauffage uniforme d'un segment de 11mm comme souhaité. Comme toujours le chauffage s'élargi par diffusion thermique mais cette fois ci de façon moindre le long de l'axe X à l'extrémité du segment, comparativement aux cartes de thermiques représentées aux figures 19a et 19b. La nécrose obtenue en fin de chauffage de $11 \times 4mm^2$ représentée figure 23c est similaire à la précédente à la différence près qu'elle est légèrement plus courte d'un millimètre.

L'étude statistique (voir figure 24) de la température minimum, moyenne et maximale de la température des 11 voxels sous contrôle est légèrement différente puisque la consigne n'est pas la même pour chacun des points. Par conséquent durant la montée en température le point central correspond systématiquement au point le plus chaud et les deux points périphériques aux points les plus froids. Pendant cette phase l'écart entre la température maximum et minimum est élargi de façon à correspondre à la différence entre la consigne centrale et la consigne la plus décalée. Une fois la montée en température finie cet écart entre la température maximum redevient de 2°C comme précédemment. De même le plateau de température à 15°C est suivi avec une précision moyenne de 0,5°C.

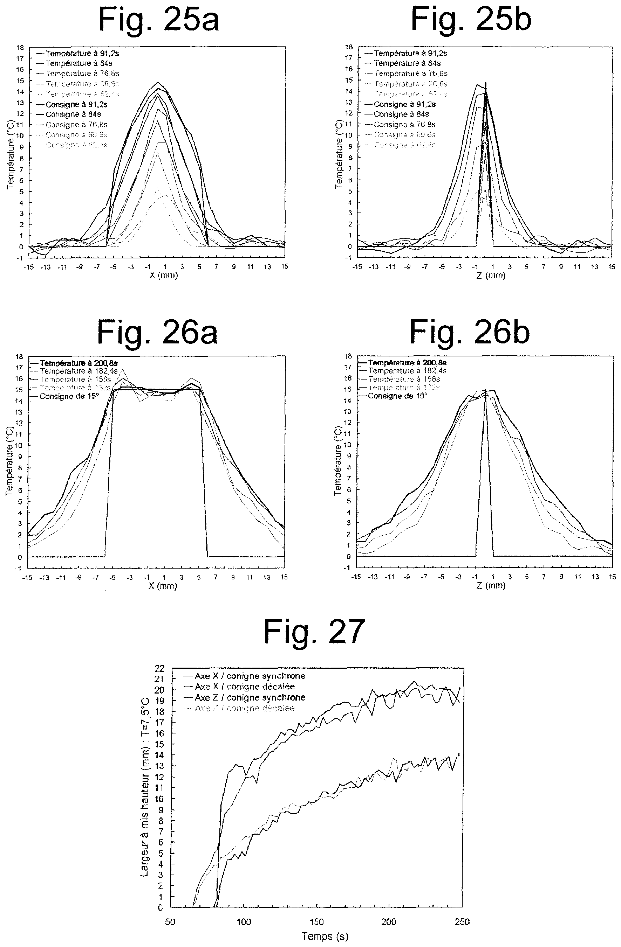

Pour observer la flexibilité avec laquelle l'algorithme d'asservissement spatial peut contrôler la température, 5 des consignes triangulaires et des températures correspondantes obtenues sont représentées aux figures 25a et 25b. La répartition spatiale de la température suit précisément la consigne de température quelque soit sa forme, tant que les gradients de température imposés ne dépassent pas ceux produit par la diffusion thermique. Dans cet exemple avec une pente maximale de 2°C/mm cette limitation n'est pas gênante. De plus la température à l'extérieur du volume chauffé le long de l'axe X est moindre puisqu'elle ne dépasse pas 4°C à l'instant 90s. L'élargissement du volume chauffé le long de l'axe Z est en revanche légèrement plus élevé que précédemment puisque la montée en température au point central est effectuée plus rapidement.

Comme le montre les figures 26a et 26b, la température est maintenue constante et homogène sur le volume chauffé, seule la température extérieure augmente progressivement. Pour évaluer cet élargissement du chauffage par diffusion thermique et comparer les deux expériences précédentes, la figure 27 reporte la largeur à mi-hauteur soit 7,5°C du volume chauffé le long des axes X et Z.

Durant la montée en température de 60s à 120s, les largeurs à mi-hauteur des deux expériences sont très différentes. Le chauffage réalisé avec une consigne de température synchronisée fait franchir la valeur de 7,5°C à l'ensemble des points simultanément, ce qui explique l'augmentation brutale de la largeur à mi-hauteur à 80s. D'une autre façon, le chauffage réalisé avec une consigne décalée amène d'abord la température centrale au dessus de 7,5°C puis ensuite les points voisins de proche en proche, ce qui induit une augmentation progressive de la largeur à mi-hauteur jusqu'à 11mm. C'est ensuite la diffusion thermique qui fait croître la largeur à mi-hauteur. Au bilan, la largeur à mi-hauteur le long de l'axe X pour le chauffage avec une consigne décalée est inférieure à celui obtenu pour une consigne synchronisée de 1mm car les points excentrés sont chauffés plus tardivement. Le long de l'axe Z, le phénomène inverse est observé de façon moindre puisque le point central est chauffé plus rapidement. Avec l'avancement du chauffage ces différences s'estompent. Ceci justifie la taille de la nécrose obtenue avec une consigne décalée qui est plus longue d'un millimètre et de largeur identique à celle obtenue avec une consigne synchronisée.

Contrôle de la température 3D cubique

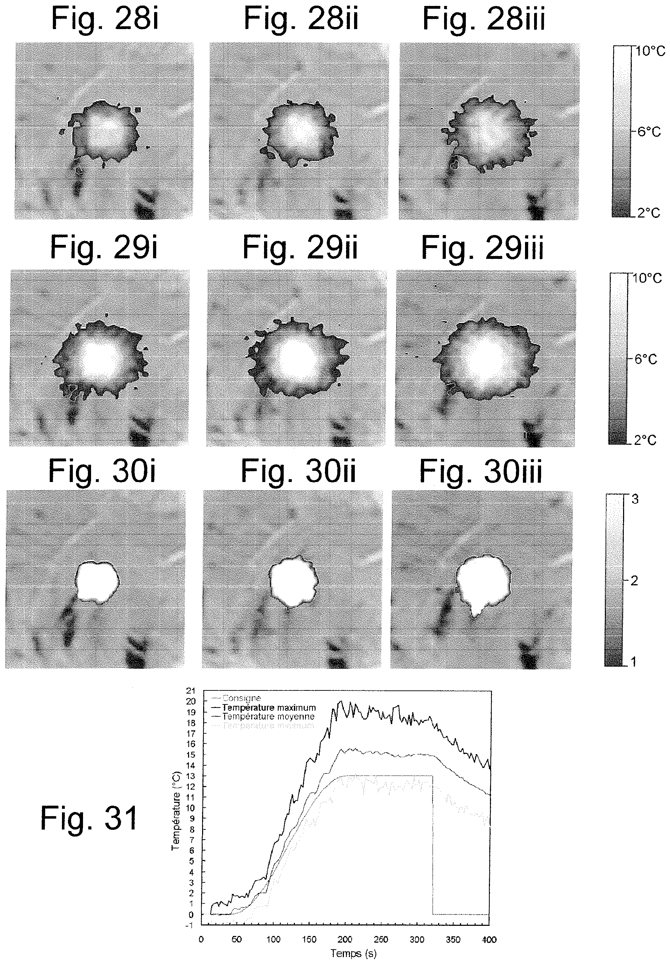

Pour évaluer les performances de l'algorithme de contrôle de température sur un volume 3D, le cube de 147 points décrit précédemment aux figures 13i, 13ii, et 13iii a été chauffé avec une consigne synchronisée de 12°C durant 220s pour obtenir une dose thermique sur l'ensemble de ce volume. Les résultats obtenus durant cette expérience sont reportés aux figures 28, 29 et 30.

Les cartographies de température des 3 coupes d'intérêt indiquent un chauffage cubique de 6°C puis de 10°C comme escompté. La forme carré apparaît distinctement sur la coupe 3 (la plus en profondeur dans le tissu) et s'arrondi de plus en plus sur les coupes 4 et 5. Il en va de même pour la nécrose obtenue en fin de chauffage qui forme un carré de coté 8mm qui s'arrondit progressivement sur les coupes 4 et 5.

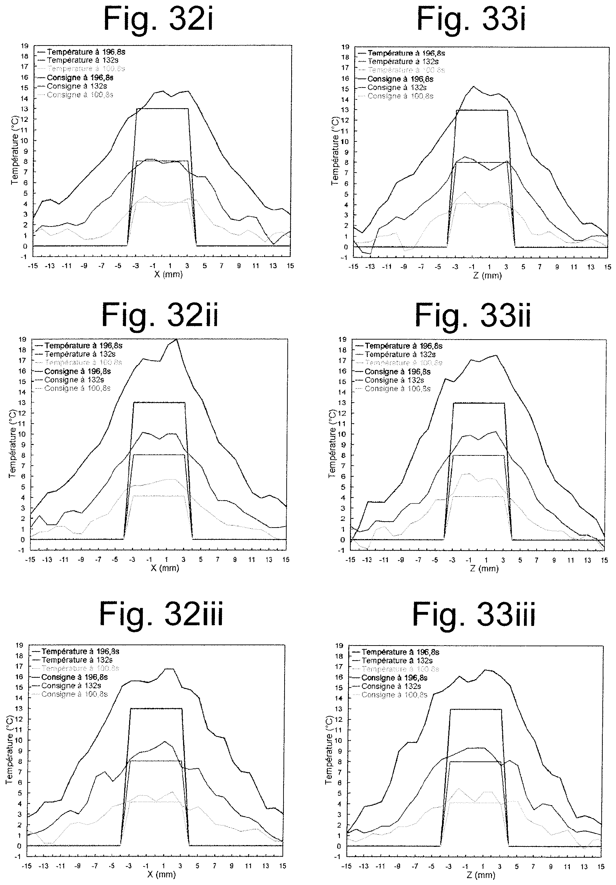

L'analyse statistique de la température minimum, moyenne et maximum sur l'ensemble du volume chauffé, représentée figure 31, indique des écarts plus importants que ceux obtenus précédemment. En effet la différence entre la température maximum et la température minimum est en moyenne de 6°C au lieu de 2°C puisqu'il y a 147/11=13 fois plus de points contrôlés. L'influence du bruit sur cette différence est par conséquent plus de 3 fois plus importante. La température moyenne calculée est par contre d'autant moins bruitée. Cependant elle indique clairement un dépassement constant de 3°C de la température de consigne. Cet effet provient des algorithmes d'asservissement PID et de détection du maximum de l'énergie requise qui forcent le voxel le plus froid à atteindre la consigne de température au dépit des voxels trop chauds. En outre le voxel de température minimum se situe systématiquement sur la coupe 3 et celui de température maximum sur la coupe 4. Pour observer cet effet plus en détail, les figures 32 et 33 montrent le chauffage réalisé sur les trois coupes centrales le long des axes X et Z respectivement, au début, au milieu et à la fin de la montée en température.

La consigne de température est atteinte précisément sur la coupe 3. Toutefois, cette coupe est de façon globale plus froide que les coupes 4 et 5. En effet, la température mesurée sur ces deux autres coupes dépasse la consigne de température. Sur la coupe 4, la plus chaude de toutes, ce dépassement peut atteindre 6°C.

De part l'effet de superposition des différents cônes de propagation ultrasonore, le chauffage d'une surface induit un chauffage 1,3 fois plus allongé. Ainsi chaque chauffage réalisé sur la coupe 3 induit un chauffage équivalent sur les coupes 4 et 5. Avec en plus l'effet d'atténuation de l'onde ultrasonore durant la traversé du tissu, l'énergie arrivant sur la coupe 3 (la plus en profondeur dans le tissu) est inférieure à celle ayant traversée les coupes 4 et 5. Au final seule la température sur la coupe 3 est contrôlée, puisqu'un chauffage sur la coupe la plus en profondeur induit un chauffage plus important sur les autres coupes. En effet comme le détaille le tableau 1, 85% de l'énergie totale émise est focalisé dans la coupe 3

*Tableau 1 : Répartition en profondeur de l'énergie déposée pour un chauffage cubique*

|  | Coupe 3 | Coupe 4 | Coupe 5 |
|---|---|---|---|
| Energie déposée | 85,25% | 8,03% | 6,72% |

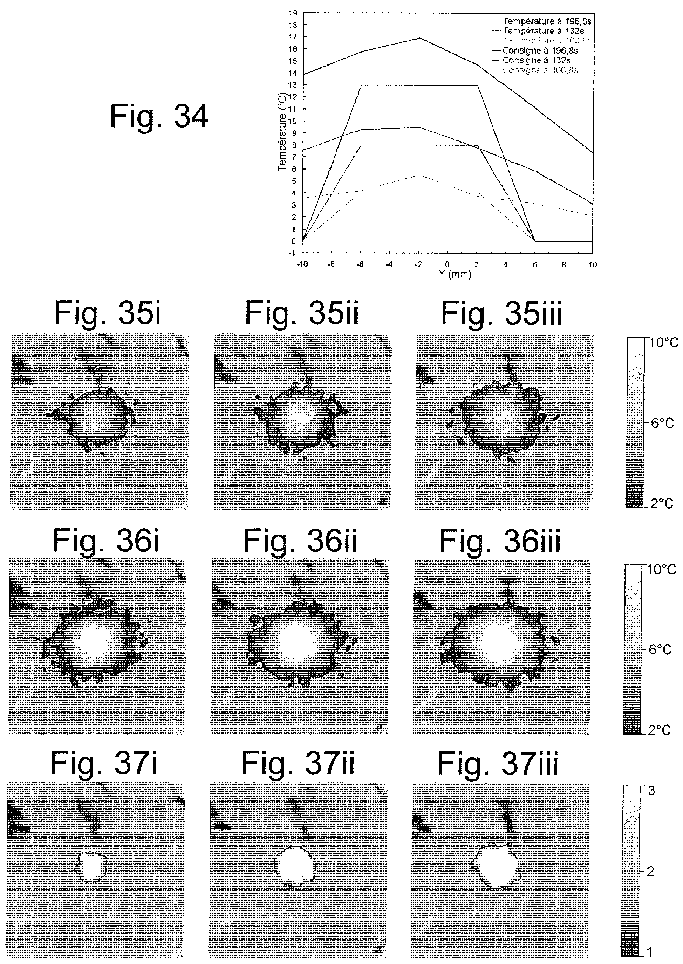

Malgré tout, comme le montre la figure 34 qui représente la température relevée le long de l'axe Y, le maximum se trouve sur la coupe 4. Aussi à l'extérieur des coupes 3, 4 et 5, la température décroît. Evidement cette décroissance reste très limitée, surtout sur la coupe 6 entre le transducteur et le volume chauffé. La nécrose obtenue sur cette coupe est de taille équivalente à celle obtenue sur les autres coupes. L'absorption du tissu et l'effet de chevauchement imposent l'obtention d'un chauffage plutôt allongé vers la source d'énergie le transducteur. En pratique cette conséquence est néfaste pour la sécurité du traitement, puisqu'il peut provoquer des brûlures cutanées si le traitement est appliqué à moins de 15mm de la peau.

En dépit de ces contraintes physiologiques et matérielles, l'asservissement PID permet de définir l'énergie strictement nécessaire pour nécroser la coupe la plus en profondeur. L'algorithme peut-être encore optimisé en utilisant un modèle de la forme du point de focalisation qui tient compte du coefficient d'absorption et d'atténuation du tissu, mais même sans cela le résultat voulu est obtenu.

*Tableau 2 : Répartition géométrique de l'énergie déposée pour un chauffage cubique*

|  | Coins | Arêtes | Autre |
|---|---|---|---|
| Energie déposée | 63% | 33,3% | 3,7% |

D'autre part l'étude de la répartition géométrique de l'énergie déposée (voir tableau 2) indique que deux tiers de l'énergie totale est situé dans les coins, un tiers sur les arêtes et un pourcentage négligeable au centre. Cette réparation est similaire à la densité de points obtenue avec l'algorithme de détection du maximum des figures 16a à 16c. De façon générale pour une topologique 3D, la focalisation requise à l'intérieur du volume est très faible puisque celui-ci est chauffé indirectement par diffusion thermique des points de focalisation périphériques. Pour cette raison l'utilisation d'une consigne de température décalée en trois dimensions ne présente aucune différence notable sur le chauffage produit.

Contrôle de la température 3D sphérique

L'obtention d'un chauffage cubique est théoriquement très instructive, mais ne correspond pas vraiment à la forme des tumeurs qui sont en général plutôt sphériques. De plus la quasi-totalité de l'énergie est déposée dans les coins et les arêtes du cube. Pour obtenir une répartition du dépôt énergétique plus homogène et un chauffage plus réaliste l'expérience précédente a été reproduite avec un chauffage sphérique. La consigne temporelle de température est identique mais les coins et les arrêtes du cubes ont été retirés de la consigne spatiale.

Similairement aux autres expériences, deux cartographies de températures obtenues au milieu et à la fin de la montée en température ainsi que la dose produite à la fin du chauffage sont reportées aux figures 35 et 36. Étant donnée la nouvelle consigne sphérique choisie, les cartes de température et de dose thermique obtenues sont arrondies de diamètres plus ou moins grands selon la coupe observée. Seul le diamètre du chauffage et de la nécrose produite sur la coupe 5 (figure 37iii) est légèrement plus grand que prévu. D'autre part, le chauffage est assez homogène sur le volume cible excepté sur la coupe 4 qui est un peu plus chaude au centre de la sphère. Comme pour chaque volume chauffé, la température centrale est plus élevée compte tenu de l'effet de diffusion thermique et de la superposition des faisceaux acoustiques.

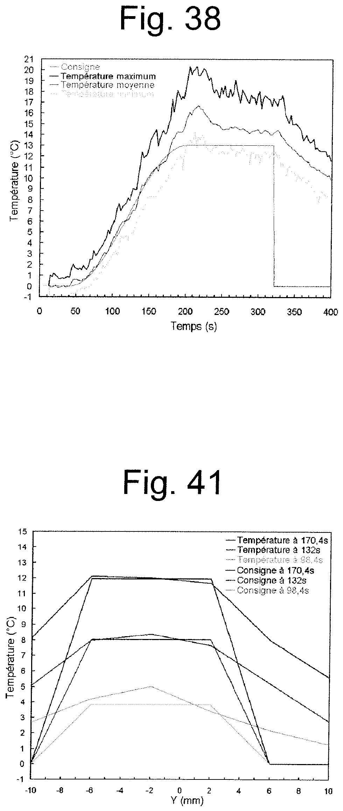

La différence entre la température maximum et minimum sur le volume asservi présentée figure 38 est maintenant de 5°C. Cet écart bien que plus faible que précédemment, reste toujours assez important car le nombre de voxel contrôlé 89 est bien plus grand que le nombre de points réalisés par trajectoire d'une part et la forme sphérique recherchée ne fait pas partie des formes rigoureusement réalisables. Les volumes réalisables avec cette géométrie de transducteur sont plutôt un ellipsoïde allongé dont le rapport de la hauteur sur la largeur doit être supérieur ou égal à 1,3. Toutefois la forme sphérique est tout de même plus réalisable que la forme cubique, et par conséquent le dépassement de la température moyenne par la température de consigne est de 2°C au lieu de 3°C précédemment.

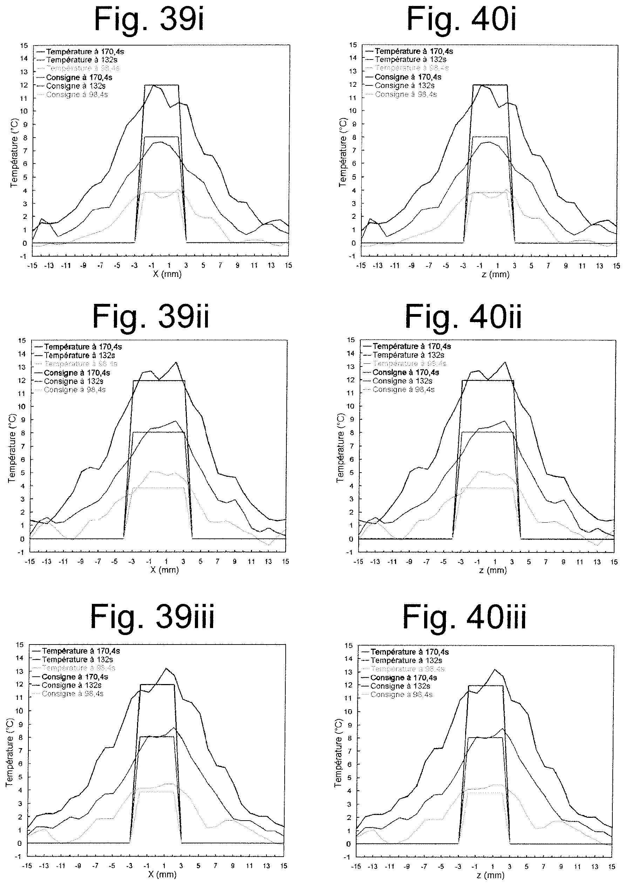

Les figures 39 et 40 montrent plus en détail les températures mesurées le long des axes X et Z pendant la montée en température. Comme précédemment la coupe 3 est la plus froide et la coupe 4 la plus chaude. Par contre le chauffage réalisé est beaucoup plus proche de la consigne de température. Bien que le tissu et le transducteur soient identiques, l'effet d'atténuation à la traversée du tissu et l'effet de superposition des cônes de propagation sont moins importants. L'atténuation est moindre car la coupe 3 la plus en profondeur comporte 21 points au lieu de 47 précédemment. Le chevauchement est plus faible puisque les points les plus éloignés du centre, les coins et les arrêtes du cube ont été retirés de la consigne spatiale. La répartition en profondeur de l'énergie déposée est maintenant plus homogène.

*Tableau 3 : Répartition en profondeur de l'énergie déposée pour un chauffage sphérique*

|  | Coupe 3 | Coupe 4 | Coupe 5 |
|---|---|---|---|
| Energie déposée | 46,88% | 49,5% | 3,62% |

Comme le détaille le tableau 3, les points de focalisations sont répartis à peu près à part égale entre la coupe 3 et la coupe 4. Ce choix conduit au chauffage voulu de la coupe 5, bien qu'il ne comporte quasiment aucun point de focalisation. Par contre cette coupe ayant été chauffée en focalisant dans les coupes supérieures, la zone qui est chauffée ne peut pas être de diamètre inférieur à celle de la coupe 4. C'est pour cette raison que la nécrose finale obtenue sur la coupe 5 est largement plus grande que celle définie sur la consigne spatiale de température.

La figure 41 indiquant la température centrale relevée le long de l'axe Y confirme la bonne homogénéité du chauffage entre les coupes 3, 4 et 5. De plus la température décroît plus rapidement sur les autres coupes puisque le volume chauffé est de dimension plus faible. D'autre part, ce n'est pas un hasard, si la température sur la coupe 3 est légèrement en dessous de la température de consigne au début de la montée en température comme précédemment sur la figure 31. Cette erreur liée à l'incomplétude de la forme du point de focalisation vis-à-vis du coefficient d'atténuation est rapidement compensée par l'algorithme d'asservissement PID.

En conclusion, lorsque le chevauchement des différents points focaux est moindre, le contrôle spatial de la température peut s'effectuer en assimilant l'énergie requise à la densité de points à appliquer. C'est notamment le cas pour le contrôle spatial 2D multispirales, puisque l'ensemble des points focaux se trouvent dans le plan coronal qui est perpendiculaire à l'axe de propagation de l'onde.

En revanche lorsque le plan d'imagerie inclut l'axe de propagation de l'onde ultrasonore, les différents points de focalisations se chevauchent.

L'approximation de l'énergie requise par la densité de points n'est alors plus valide puisqu'elle produit une énergie trop élevée. Ce dépassement de l'énergie produite étant très fortement prononcé lors un contrôle 3D de la température, il est indispensable de prendre en compte la forme du point de focalisation pour quantifier l'effet de chevauchement.

En associant l'algorithme de détection du maximum avec l'asservissement spatial PID pour prendre en compte la forme du point focal, le contrôle de la température est alors réalisable en 3D. La température peut ainsi être contrôlée sur l'ensemble du volume traité.

Le système de rétroaction 3D décrit a pour but de contrôler la température minimum plutôt que la température moyenne du volume de consigne. Ceci est pratique pour effectuer l'ablation d'une tumeur maligne puisque la nécrose est ainsi garantie sur chaque voxel du volume. En revanche pour une utilisation non destructrice comme l'activation de liposome thermosensible, cet asservissement devrait être modifié pour assurer plutôt une température maximale à ne pas dépasser. L'idéal étant bien sûr un asservissement qui réalise les deux simultanément. Cela est plus ou moins réalisable selon la taille et la forme du volume chauffé. Par exemple un segment de quelques points s'en approche assez bien. Pour obtenir un contrôle précis de tout un volume quelque soit sa forme, l'idéal est d'avoir un transducteur avec un grand angle d'ouverture pour diminuer l'effet de chevauchement. Cette limitation technologique bien qu'améliorable ne sera jamais parfaite. Il est donc recommandé avant de commencer un traitement d'effectuer une simulation du chauffage induit pour anticiper et ajuster le volume de l'ablation.

Le lecteur aura compris que de nombreuses modifications peuvent être apportées sans sortir matériellement des nouveaux enseignements et des avantages décrits ici. Par conséquent, toutes les modifications de ce type sont destinées à être incorporées à l'intérieur de la portée du dispositif traitement thermique d'un tissu biologique selon l'invention, et du procédé de traitement thermique associé.

## Revendications

1. Dispositif de traitement thermique d'une région cible d'un tissu biologique comprenant :

   - des moyens générateurs d'énergie (3,4) pour fournir de l'énergie en un point focal (P) dans la région cible,
   - des moyens (2) pour mesurer la distribution spatiale de température dans ladite région,
   - une unité de contrôle (7) comprenant des moyens pour commander le déplacement du point focal (P) vers des points de traitement successifs et des moyens pour commander l'énergie à fournir par les moyens générateurs (3,4) vers les points de traitement successifs,

   caractérisé en ce que l'unité de contrôle (7) comprend en outre des moyens pour déterminer une répartition spatiale et énergétique des points de traitement à effectuer,
   caractérisé en ce que ces moyens sont agencés pour déterminer la répartition spatiale et énergétique des points de traitement à effectuer en fonction d'une répartition spatiale d'énergie requise définie par un système de régulation de la température pour traiter la région cible, un point de traitement étant déterminé comme le point où une répartition spatiale d'énergie restante pour le traitement de la région cible présente un maximum, la répartition spatiale d'énergie restante correspondant à la répartition spatiale d'énergie requise pour traiter la région cible retranchée des répartitions spatiales d'énergie caractéristiques des points de traitement antérieurement déterminés.

2. Dispositif selon la revendication 1, caractérisé en ce que la répartition spatiale des points de traitement est tridimensionnelle.

3. Dispositif selon l'une quelconque des revendications 1 et 2, **caractérisé en ce que** l'unité de contrôle comprend des moyens pour déterminer la répartition spatiale d'énergie requise pour traiter la région cible selon un système de régulation Proportionnel-Intégral-Dérivé.

4. Dispositif selon l'une quelconque des revendications 1 à 3, **caractérisé en ce que** les moyens pour déterminer la répartition spatiale et énergétique des points de traitement à effectuer comprennent des moyens de déconvolution de la répartition spatiale d'énergie requise pour traiter la région cible par une répartition spatiale d'énergie caractéristique d'un point de traitement ponctuel.

5. Dispositif selon l'une quelconque des revendications 1 à 4, **caractérisé en ce que** l'unité de contrôle comprend des moyens pour déterminer la répartition spatiale et énergétique des points de traitement à effectuer tel que :

$$E^0(\vec{r}) = E^{REQUISE}(\vec{r}) \quad \text{et} \quad \Gamma^0(\vec{r}) = 0$$

$$\left| \begin{array}{l} \text{soit } \vec{r}_n \text{ tel que } E^n(\vec{r}_n) = \max(E^n(\vec{r})) \\ E^{n+1}(\vec{r}) = E^n(\vec{r}) - R \cdot E^n(\vec{r}_n) \cdot E^{1pt}(\vec{r} - \vec{r}_n) \\ \Gamma^{n+1}(\vec{r}) = \Gamma^n(\vec{r}) + R \cdot E^n(\vec{r}_n) \cdot \delta(\vec{r} - \vec{r}_n) \end{array} \right.$$

où $E^i(\vec{r})$ correspond à la différence entre la répartition spatiale d'énergie requise et les répartitions spatiales d'énergie caractéristiques des $i$ points de traitement $\vec{r}_i$ antérieurs, $\Gamma^i(\vec{r})$ correspond à la répartition spatiale des points de traitement associés, $\delta$ est la fonction de Dirac qui est nulle en tout point $\vec{r}$ différent de l'origine où elle vaut 1, $R$ est un pourcentage choisi de façon arbitraire pour que la répartition spatiale d'énergie caractéristique d'un point de traitement soit inférieure à la répartition spatiale d'énergie requise $E^{REQUISE}(\vec{r})$.

6. Dispositif selon l'une quelconque des revendications 1 à 5, **caractérisé en ce que** l'unité de contrôle comprend des moyens pour contrôler l'énergie fournie par les moyens générateurs d'énergie en fonction d'une consigne de température non uniforme.

7. Dispositif selon l'une quelconque des revendications 1 à 6, **caractérisé en ce que** l'unité de contrôle comprend des moyens pour contrôler l'énergie fournie par les moyens générateurs d'énergie en fonction d'une consigne de température décalée temporellement pour chaque point de traitement.


**Patentansprüche**

1. Vorrichtung zur Wärmebehandlung einer Zielregion eines biologischen Gewebes, Folgendes umfassend:

   - Energieerzeugungsmittel (3, 4) zum Bereitstellen von Energie in einem Brennpunkt (P) in der Zielregion,
   - Mittel (2) zum Messen der räumlichen Temperaturverteilung in der besagten Region,
   - eine Steuereinheit (7), Mittel zum Steuern der Bewegung des Brennpunkts (P) zu aufeinanderfolgenden Behandlungsstellen und Mittel zum Steuern der Energie umfassend, die durch die Erzeugungsmittel (3, 4) für die aufeinanderfolgenden Behandlungsstellen beizustellen ist,

   **dadurch gekennzeichnet, dass** die Steuereinheit (7) darüber hinaus Mittel zur Bestimmung einer räumlichen und energetischen Verteilung der Stellen für die durchzuführende Behandlung umfasst,
   **dadurch gekennzeichnet, dass** die Mittel angeordnet sind, um die räumliche und energetische Verteilung der Stellen für die durchzuführende Behandlung in Abhängigkeit von einer nötigen räumlichen Energieverteilung zu bestimmen, die durch ein System zum Regeln der Temperatur zur Behandlung der Zielregion definiert wird, wobei eine Behandlungsstelle als jene Stelle bestimmt wird, wo eine verbleibende räumliche Energieverteilung für die Behandlung der Zielregion einen Höchstwert aufweist, wobei die verbleibende räumliche Energieverteilung der nötigen räumlichen Energieverteilung zur Behandlung der Zielregion abzüglich der charakteristischen räumlichen Energieverteilungen jener Behandlungsstellen entspricht, die zuvor bestimmt worden sind.

**2.** Vorrichtung nach Anspruch 1, **dadurch gekennzeichnet, dass** die räumliche Verteilung der Behandlungsstellen dreidimensional ist.

**3.** Vorrichtung nach irgendeinem der Ansprüche 1 und 2, **dadurch gekennzeichnet, dass** die Steuereinheit Mittel zum Bestimmen der nötigen räumlichen Energieverteilung zur Behandlung der Zielregion gemäß einem Proportional-Integral-Differential-Regelungssystem umfasst.

**4.** Vorrichtung nach irgendeinem der Ansprüche 1 bis 3, **dadurch gekennzeichnet, dass** die Mittel zum Bestimmen der räumlichen und energetischen Verteilung der Stellen für die durchzuführende Behandlung Mittel zum Dekonvolutionieren der nötigen räumlichen Energieverteilung für die Behandlung der Zielregion durch eine charakteristische räumliche Energieverteilung einer punktuellen Behandlungsstelle enthalten.

**5.** Vorrichtung nach irgendeinem der Ansprüche 1 bis 4, **dadurch gekennzeichnet, dass** die Steuereinheit Mittel zur Bestimmung der räumlichen und energetischen Verteilung der Stellen für die durchzuführende Behandlung umfasst, wie:

$$E^0(\vec{r}) = E^{N\ddot{O}TIG}(\vec{r}) \; und \; \Gamma^0(\vec{r}) = 0$$

$$oder \; \vec{r_n} \; wie \; E^n(\vec{r_n}) = \max \left( E^n(\vec{r_n}) \right)$$

$$E^{n+1}(\vec{r}) = E^n(\vec{r}) - R * E^n(\vec{r_n}) * E^{1\,St}(\vec{r} - \vec{r_n})$$

$$\Gamma^{n+1}(\vec{r}) = \Gamma^n(\vec{r}) + R * E^n(\vec{r_n}) * \delta(\vec{r} - \vec{r_n})$$

wobei $E^i(\vec{r})$ der Differenz zwischen der nötigen räumlichen Energieverteilung und den charakteristischen räumlichen Energieverteilungen der $i$ vorherigen Behandlungsstellen $\vec{r_i}$ entspricht, $\Gamma^i(\vec{r})$ der räumlichen Verteilung der zugeordneten Behandlungsstellen entspricht, $\delta$ die Dirac-Funktion ist, die an jeder Stelle $\vec{r}$ ungleich dem Ausgangspunkt Null ist, wo sie 1 ist, $R$ ein zufällig ausgewählter Prozentsatz ist, damit die charakteristische räumliche Energieverteilung einer Behandlungsstelle kleiner ist, als die nötige räumliche Energieverteilung $E^{N\ddot{O}TIG}(\vec{r})$.

**6.** Vorrichtung nach irgendeinem der Ansprüche 1 bis 5, **dadurch gekennzeichnet, dass** die Steuereinheit Mittel zum Steuern der Energie umfasst, die durch die Energieerzeugungsmittel in Abhängigkeit von einem nicht einheitlichen Temperatursollwert bereitgestellt wird.

**7.** Vorrichtung nach irgendeinem der Ansprüche 1 bis 6, **dadurch gekennzeichnet, dass** die Steuereinheit Mittel zum Steuern der Energie umfasst, die durch die Energieerzeugungsmittel in Abhängigkeit von einem vorübergehend verschobenen Temperatursollwert für jede Behandlungsstelle bereitgestellt wird.

**Claims**

**1.** Device for the heat treatment of a target region of a biological tissue, comprising:

  - energy generating means (3, 4) for supplying energy to a focal point (P) in the target region,
  - means (2) for measuring the spatial temperature distribution in said region,
  - a control unit (7) comprising means for controlling the displacement of the focal point (P) towards successive treatment points and means for controlling the energy to be supplied by the generating means (3, 4) to the successive treatment points,

**characterised in that** the control unit (7) further includes means for determining a spatial and energy distribution of the treatment points where treatment is to be carried out,
**characterised in that** these means are arranged to determine the spatial and energy distribution of the treatment points where treatment is to be carried out according to a spatial distribution of energy required, defined by a temperature control system to treat the target region, a treatment point being determined as being the point at which a spatial distribution of remaining energy for treating the target region is at its maximum, the spatial distribution of remaining energy corresponding to the spatial distribution of energy required to treat the target region subtracted from the spatial energy distributions characteristic of the previously determined treatment points.

**2.** Device according to claim 1, **characterised in that** the spatial distribution of the treatment points is three-dimensional.

**3.** Device according to either one of claims 1 and 2, **characterised in that** the control unit comprises means for determining the spatial distribution of energy required for treating the target region according to a Proportional-Integral-Derivative control system.

**4.** Device according to any one of claims 1 to 3, **characterised in that** the means for determining the spatial and energy distribution of the treatment points where treatment is to be carried out comprise deconvolution means to deconvolve the spatial distribution of energy required to treat the target region by a spatial energy distribution characteristic of an isolated treatment point.

**5.** Device according to any one of claims 1 to 4, **characterised in that** the control unit comprises means for determining the spatial and energy distribution of the treatment points where treatment is to be carried out, such that:

$$E^0(\vec{r}) = E^{REQUIRED}(\vec{r}) \; and \; \Gamma^0(\vec{r}) = 0$$

$$thus \; \vec{r_n} \; such \; that \; E^n(\vec{r_n}) = \max \left( E^n(\vec{r_n}) \right)$$

$$E^{n+1}(\vec{r}) = E^n(\vec{r}) - R * E^n(\vec{r_n}) * E^{1\,St}(\vec{r} - \vec{r_n})$$

$$\Gamma^{n+1}(\vec{r}) = \Gamma^n(\vec{r}) + R * E^n(\vec{r_n}) * \delta(\vec{r} - \vec{r_n})$$

where $E^i(\vec{r})$ corresponds to the difference between the spatial distribution of energy required and the spatial energy distributions characteristic of the previous i treatment points $\vec{r_i}$, $\Gamma^i(\vec{r})$ corresponds to the spatial distribution of the associated treatment points, $\delta$ is the Dirac delta function which is exactly zero r and different from the original value of 1, $R$ is a percentage chosen in an arbitrary manner such that the spatial energy distribution characteristic of a treatment point is less than the spatial distribution of energy required $E^{REQUIRED}(\vec{r})$.

**6.** Device according to any one of claims 1 to 5, **characterised in that** the control unit comprises means for controlling the energy supplied by the energy generating means according to a non-uniform temperature set-point.

**7.** Device according to any one of claims 1 to 6, **characterised in that** the control unit comprises means for controlling the energy supplied by the energy generating means according to a temperature set-point offset in time for each treatment point.

# Fig. 1

# Fig. 2a  Fig. 2b  Fig. 2c

# Fig. 3

# Fig. 4a

# Fig. 4b

# Fig. 5

# Fig. 6a

# Fig. 6b

# Fig. 6c

# Fig. 7

# Fig. 8a

# Fig. 8b

# Fig. 8c

# Fig. 9

# Fig. 10a

# Fig. 10b

# Fig. 10c

# Fig. 11

Fig. 12i    Fig. 12ii    Fig. 12iii

Fig. 13i    Fig. 13ii    Fig. 13iii

Fig. 14i    Fig. 14ii    Fig. 14iii

Fig. 15i    Fig. 15ii    Fig. 15iii

# Fig. 16a  Fig. 16b  Fig. 16c

# Fig. 17

# Fig. 18a  Fig. 18b

# Fig. 19a　　Fig. 19b　　Fig. 19c

# Fig. 20

# Fig. 21a　　　Fig. 21b

# Fig. 22a

# Fig. 22b

# Fig. 23a

# Fig. 23b

# Fig. 23c

# Fig. 24

EP 2 056 937 B1

# Fig. 25a

# Fig. 25b

# Fig. 26a

# Fig. 26b

# Fig. 27

# Fig. 28i

# Fig. 28ii

# Fig. 28iii

# Fig. 29i

# Fig. 29ii

# Fig. 29iii

# Fig. 30i

# Fig. 30ii

# Fig. 30iii

# Fig. 31

## Fig. 32i

## Fig. 33i

## Fig. 32ii

## Fig. 33ii

## Fig. 32iii

## Fig. 33iii

## Fig. 34

## Fig. 35i    Fig. 35ii    Fig. 35iii

## Fig. 36i    Fig. 36ii    Fig. 36iii

## Fig. 37i    Fig. 37ii    Fig. 37iii

## Fig. 38

## Fig. 41

# Fig. 39i

# Fig. 40i

# Fig. 39ii

# Fig. 40ii

# Fig. 39iii

# Fig. 40iii

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 0404562
- WO 0243804 A

- FR 9911418